# EUROPEAN PATENT SPECIFICATION

(11) **EP 4 558 124 B1**
(45) Date of publication and mention of the grant of the patent: **30.09.2026**
(21) Application number: 23744477.3
(22) Date of filing: 19.07.2023
(51) Int. Cl.: A61K 9/00, A61K 9/10, A61K 31/18, A61K 47/02, A61K 47/26, A61K 47/32

(54) **POWDER MIXTURE COMPRISING REPARIXIN**
REPARIXIN-HALTIGE PULVERMISCHUNG
MÉLANGE DE POUDRE COMPRENANT DE LA RÉPARIXINE

(30) Priority: 19.07.2022 EP 22185649
(43) Date of publication of application: 28.05.2025
(73) Proprietor: Dompé farmaceutici SpA, 20122 Milano (IT)
(72) Inventor: ROMEO, Tiziana, 67100 L'Aquila (IT); ALBERTINI, Barbara, 67100 L'Aquila (IT); DRAGANI, Maria Concetta, 67100 L'Aquila (IT)
(74) Representative: Dompé farmaceutici Spa
(86) International application number: PCT/EP2023/070011
(87) International publication number: WO 2024/017945

(56) References cited:
- US-A1- 2019 224 148
- US-A1- 2020 222 420
- DOMPÉ FARMACEUTICI S.P.A ET AL: "Reparixin in COVID-19 Pneumonia -Ecacy and Safety Study record dates Study Registration Dates Results Reporting Dates", 24 May 2022 (2022-05-24), https://beta.clinicaltrials.gov/study/NCT04794803https://beta.clinicaltrials.gov/study/NCT04794803, XP093006143, Retrieved from the Internet <URL:https://beta.clinicaltrials.gov/study/NCT04794803> [retrieved on 20221208]

## Description

### FIELD OF THE INVENTION

The present invention relates to a powder mixture comprising reparixin, which is suitable to produce a reconstituted liquid suspension at the time of use.

### STATE OF THE ART

Reparixin, also known as R-(-)-2-(4-isobutylphenyl)propionyl methansulfonamide and repertaxin, is a non competitive allosteric inhibitor of the interleukin 8 (IL-8) receptors, CXCR1 and CXCR2 (Bertini R, et al., Proc Natl Acad Sci. 2004;101(32):11791-6). Reparixin has a therapeutic potential in preventing or treating pathologies where the activation of this pathway is detrimental.

For instance, IL-8 is involved in neutrophil infiltration in pathologies such as psoriasis (B. J. Nickoloff et al., Am. J. Pathol., 1991, 138, 129), rheumatoid arthritis (M. Selz et al., J. Clin. Invest. 1991, 87, 463), ulcerative colitis (Y. R. Mahkla et al., Clin. Sci., 1992, 82, 273), acute respiratory distress syndrome (ARDS), idiopathic fibrosis (P. C. Carré et al., J. Clin. Invest,. 1991, 88, 1802, and E.J. Miller et al., Am. Rev. Respir. Dis. 1992, 146:427-432) and glomerulonephritis (T. Wada et al., J. Exp. Med., 1994, 180,1135).

In vitro and preclinical small animal studies have demonstrated that binding of reparixin to CXCR1/CXCR2 can prevent leukocyte recruitment and activation of inflammation. Action of reparixin includes inhibition of neutrophil stimulation and downstream effects of neutrophils, such as NETosis, the formation of NETs, during an inflammatory response (Cheng OZ, Palaniyar N., Front Immunol.2013;4:1).

The dose, safety, and pharmacokinetics of reparixin have been investigated in clinical studies of patients with metastatic breast cancer (Schott AF, et al., Clin Cancer Res. 2017;23(18):5358-65; Goldstein LJ et al., Breast Cancer Res Treat. 2021;190(2):265-75).

A phase 2, multicenter, open-label, randomized, clinical trial to assess the efficacy and safety of reparixin compared with the available treatments considered as standard of care (SOC) in hospitalized patients with severe COVID-19 pneumonia was carried out from May 5, 2020 until November 27, 2020, showing that treatment with reparixin led to improved clinical outcomes in patients with severe COVID-19 pneumonia compared with the standard of care (Landoni G. et al., Infect Dis Ther 2022, 26: 1-16).

At the moment, reparixin has been formulated only in form of tablets for oral administration or solution for i.v. administration. However, these types of administrations are invasive and/or are not suitable for achieving satisfactory patient compliance, especially in case of pediatric patients or patients who are unable to swallow solid tablets. Consequently, there is a need to develop new pharmaceutical dosage forms containing reparixin that are able to overcome these problems and can be administered to subjects of all conditions and ages. To this aim, suspensions are ideal candidates.

Suspensions are pharmaceutical dosage forms with two phases: an external liquid phase, also known as continuous phase or dispersion medium, and an internal phase, also known as dispersed phase, that contains particulate matter insoluble in the external phase. Suspensions are recognized as heterogeneous dispersed systems and most pharmaceutical suspensions have an aqueous dispersion medium.

Suspensions can be sold either in form of ready-to-use liquid suspensions or as powder mixtures to be reconstituted in an appropriate liquid vehicle at the time of use. Such powder mixtures generally contain the active pharmaceutical ingredient (API) and suitable suspending and dispersing agents to be diluted and agitated with a specified amount of vehicle, which is usually an aqueous vehicle.

The formulation and dispensation of this type of dosage forms is associated with several advantages: efficient dispense of hydrophobic drugs; avoidance of the use of cosolvents; masking of unpleasant taste; offering resistance to degradation of drugs due to hydrolysis, oxidation or microbial activity; easy ingesting for young or elderly patients. In addition, higher concentration of drugs can be incorporated in suspensions than in solutions (Doye, P. et al., Int J Curr Pharm Sci 2017, 9, 8).

Suspensions are thermodynamically unstable systems due to the free energy of their internal phase, which produces particle aggregation and sedimentation in order to decrease the energy of the system.

The main challenge in the development of powder mixtures which can be suspended in an appropriate vehicle at the time of use, so as to obtain a liquid suspension after shaking manually, is to ensure a rapid dispersion at the time of reconstitution in water. Also, the powder must be flowable, and the API must be uniformly dispersed in the powder mixture, and physical stability and re-dispersibility have to be assured preferably for at least one month after reconstitution.

In order to achieve the above-mentioned properties, several aspects must be considered when formulating powder mixtures suitable to produce reconstituted liquid suspensions at the time of use.

The preparation of suspensions containing reparixin is particularly difficult due to the specific features of this active pharmaceutical ingredient (API).

First of all, reparixin is produced in form of powder composed of particles having large particle size. Therefore, potential suspensions containing reparixin are coarse suspensions, i.e., suspensions characterized by an internal phase with particle size greater than ~0.5 µm (Edman, P., Journal of Aerosol Medicine 1994, 7, S-3-S-6).

In case of coarse suspensions, the internal phase with the API tends to settle under gravitational force and cause sedimentation phenomena due to its particle size. The increase of zeta potential in terms of absolute value produces an increase of the sedimentation rate. This suggests that sedimentation is driven by gravitational forces rather than by electrostatic interactions, which are insufficient to provide stability and kinetic stabilization because of the particle size of the internal phase. For this reason, the zeta potential value of coarse suspensions is usually set around zero for ensuring physical stability. This value of zeta potential corresponds to a condition where particles are surrounded by a hydration layer which hides their charge and, in case the particles aggregate and sediment, is able to prevent the formation of a compact sediment and instead promotes the formation of a loose sediment that can be resuspended easily by shaking manually. For adjusting the zeta potential value of suspensions, ionic or nonionic surfactants and different type of electrolytes (e.g. salts) are commonly employed. For instance, PEG (polyethylene glycol) is included in many commercial suspensions for this purpose.

Furthermore, reparixin has low wettability, which complicates its formulation into a powder mixture suitable to produce a reconstituted liquid suspension at the time of use. In fact, the wettability of the dispersed particles is important for ensuring homogeneity and content uniformity (Kwok, D.Y. et al., Advances in Colloid and Interface Science 1999, 81, 167-249). Wettability is the tendency of a fluid to spread on or adhere to a solid surface when they are immiscible; the consequence of a poor wettability of a powder solid is its floating on the surface of liquid medium. A poor wettability of the internal phase is mainly due to the high interfacial tension between the dispersed particles and the dispersion medium and the reduction of interfacial tension increases the stability of the suspension. When formulating a suspension or a powder mixture to be reconstituted in an aqueous vehicle at the time of use, a common way to improve the wettability of solids is the use of wetting agents such as surfactants, able to reduce the solid-liquid interfacial tension.

Another requirement necessary for ensuring stability of suspensions is the presence of so-called yield stress (τ0), which is a material property and can be defined as the stress corresponding to the yield point at which the material begins to flow or deform plastically. In light of the above, there is clearly a need to develop powder mixtures containing reparixin that, when reconstituted in an aqueous vehicle at the time of use, produce suspensions with satisfactory features. However, this is particularly challenging for this API, in view of the reasons discussed above.

### SUMMARY OF THE INVENTION

The present inventors have developed powder mixtures containing reparixin that are suitable to produce reconstituted liquid suspensions having satisfactory features in terms of stability, wettability and homogeneity of API distribution. The reconstitution is made in an aqueous vehicle at the time of use.

Specifically, the inventors have surprisingly found that the type and amount of sweetener included in the powder mixture to mask the unpleasant taste of reparixin influences the zeta potential value and the rheological properties of the liquid suspension reconstituted from such mixture and, as a consequence, its stability.

Furthermore, the inventors have found that the use of polyvinylpyrrolidone or poloxamer 188 as wetting agent allows to obtain a satisfactory wettability of reparixin. The selected wetting agent guarantees a homogeneous dispersion of reparixin in the aqueous vehicle. The inventors have further identified the types and ranges of viscosity agent and colloidal silica that must be included in the powder mixture in order to obtain a flowable powder and a stable liquid suspension of reparixin after reconstitution of the powder in the aqueous vehicle.

Accordingly, a first object of the present invention is a powder mixture comprising:
- reparixin in an amount between 3.5% w/w and 28.2% w/w,
- a viscosity agent selected from xanthan gum in an amount between 0.3% w/w and 0.5% w/w, gum arabic in an amount between 0.3% w/w and 0.5% w/w, and Avicel CL-611 in an amount between 3% w/w and 8.3% w/w,
- sucrose in an amount between 63% w/w and 89% w/w,
- a wetting agent selected from polyvinylpyrrolidone in an amount between 3% w/w and 5% w/w, and poloxamer 188 in an amount between 0.5% w/w and 1.5% w/w,
- colloidal silica in an amount between 0.7% w/w and 1.4% w/w,
with the proviso that when said viscosity agent is Avicel CL-611, said wetting agent is not polyvinylpyrrolidone.

A further object of the present invention is a process to produce a liquid suspension comprising reparixin including the step of suspending the powder mixture according to the first object of the invention in an aqueous vehicle, preferably water.

A further object of the present invention is a powder mixture according to the first object of the present invention or a reconstituted liquid suspension thereof for use in the treatment a disease or condition selected from psoriasis, rheumatoid arthritis, ulcerative colitis, acute respiratory distress syndrome (ARDS), idiopathic fibrosis, glomerulonephritis, COVID-19, pneumonia caused by SARS-CoV-2 or a variant thereof. A further object of the present invention is a powder mixture according to the first object of the invention or a reconstituted liquid suspension thereof for use in the prevention of diabetes or in delaying the onset and the progression of diabetes.

A further object of the present invention is a powder mixture according to the first object of the invention or a reconstituted liquid suspension thereof for use in the prevention and/or treatment of seizures.

A further object of the present invention is a kit comprising:
a) a powder mixture according to the first object of the invention, and
b) an aqueous vehicle, preferably water.

### DEFINITIONS

The term "antifoaming agent" as used herein refers to a chemical additive that reduces and/or hinders the formation of foam in liquid formulations, e.g., liquid suspensions, by decreasing the liquid-air surface tension.

The term "wetting agent" as used herein refers to excipients able to reduce the API-water interfacial tension by improving the wettability and spreadability of the suspended API.

As used herein with reference to a component of the powder mixture according to the invention, "% w/w" indicates the grams of that component in 100 grams of the powder mixture. For instance, the wording "reparixin in an amount of 10.16% w/w" indicates that 10.16 grams of reparixin are present in 100 grams of the powder mixture.

As used herein with reference to a component of a liquid suspension, "%w/w" indicated the grams of that component in 100 grams of the liquid suspensions. For instance, the wording "reparixin in an amount of 5% w/w" indicates 5 grams of reparixin are present in 100 grams of the liquid suspension.

As used herein, the terms "reconstituted liquid suspension" or "reconstituted liquid suspension thereof" with reference to a powder mixture indicates the liquid suspension that is produced when the powder mixture is suspended in an aqueous vehicle, preferably water.

As used herein, the term "aqueous vehicle" refers to an aqueous solvent suitable for suspending the powder mixture according to the first object of the invention. Preferably, said aqueous vehicle is water.

As used herein, the term "Avicel CL-611" indicates a spray-dried blend of microcrystalline cellulose and sodium carboxymethylcellulose, wherein sodium carboxymethylcellulose is present in an amount between 11.3% w/w and 18.8% w/w.

It is understood that terms as "suspending" and "dispersing", or "suspended" and "dispersed" can be used interchangeably in the context of the present description.

### DETAILED DESCRIPTION OF THE INVENTION

As will be explained more in details in the experimental section, the inventors have identified a quali-quantitative composition of excipients that must be included in a powder mixture comprising reparixin so that a homogeneous reconstituted liquid suspension which is stable for an adequate amount of time is obtained when the powder mixture is suspended in an aqueous vehicle.

In particular, it was unexpectedly found that the type and amount of sweetener included in the powder mixture of reparixin to mask the unpleasant taste of this API affect the zeta potential value and the rheological properties of the corresponding reconstituted liquid suspension and, therefore, its stability.

Also, the inventors have selected polyvinylpyrrolidone or poloxamer 188 as wetting agents to be included in the powder mixture in order to achieve a satisfactory wettability of the API and to ensure a homogeneous distribution in the reconstituted liquid suspension.

The reconstituted liquid suspension must be stable for at least one month after reconstitution and, in case a sediment is formed, said sediment must be easily resuspendible by manual shaking.

Accordingly, a first object of the invention is a powder mixture comprising:
- reparixin in an amount between 3.5% w/w and 28.2% w/w,
- a viscosity agent selected from xanthan gum in an amount between 0.3% w/w and 0.5% w/w, gum arabic in an amount between 0.3% w/w and 0.5% w/w, and Avicel CL-611 in an amount between 3% w/w and 8.3% w/w,
- sucrose in an amount between 63% w/w and 89% w/w,
- a wetting agent selected from polyvinylpyrrolidone in an amount between 3% w/w and 5% w/w, and poloxamer 188 in an amount between 0.5% w/w and 1.5% w/w,
- colloidal silica in an amount between 0.7% w/w and 1.4% w/w,
with the proviso that when said viscosity agent is Avicel CL-611, said wetting agent is not polyvinylpyrrolidone.

Reparixin (CAS No.: 266359-83-5), which is also known as R-(-)-2-(4-isobutylphenyl)propionyl methansulfonamide or repertaxin, is the compound having the following chemical structure:

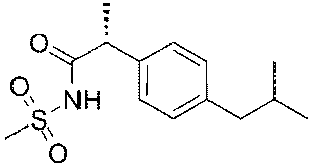

In a preferred embodiment, the powder mixture according to the first object of the invention consists for at least 98%, preferably for at least 99% of its total weight, of:
- reparixin in an amount between 3.5% w/w and 28.2% w/w,
- a viscosity agent selected from xanthan gum in an amount between 0.3% w/w and 0.5% w/w, gum arabic in an amount between 0.3% w/w and 0.5% w/w, and Avicel CL-611 in an amount between 3% w/w and 8.3% w/w,
- sucrose in an amount between 63% w/w and 89% w/w,
- a wetting agent selected from polyvinylpyrrolidone in an amount between 3% w/w and 5% w/w and poloxamer 188 in an amount between 0.5% w/w and 1.5% w/w,
- colloidal silica in an amount between 0.7% w/w and 1.4% w/w,
- a pH adjuster, preferably in an amount between 0.1% w/w and 0.2% w/w, and/or a preservative agent, preferably in an amount between 0.1% w/w and 0.5% w/w,
with the proviso that when said viscosity agent is Avicel CL-611, said wetting agent is not polyvinylpyrrolidone.

In a preferred embodiment, the powder mixture according to the first object of the invention comprises:
- reparixin in an amount between 3.5% w/w and 28.2% w/w,
- Avicel CL-611 in an amount between 3% w/w and 8.3% w/w,
- sucrose in an amount between 63% w/w and 89% w/w,
- poloxamer 188 in an amount between 0.5% w/w and 1.5% w/w,
- colloidal silica in an amount between 0.7% w/w and 1.4% w/w.

In a preferred embodiment, the powder mixture according to the first object of the invention consists for at least 98%, preferably for at least 99% of its total weight, of:
- reparixin in an amount between 3.5% w/w and 28.2% w/w,
- Avicel CL-611 in an amount between 3% w/w and 8.3% w/w,
- sucrose in an amount between 63% w/w and 89% w/w,
- poloxamer 188 in an amount between 0.5% w/w and 1.5% w/w,
- colloidal silica in an amount between 0.7% w/w and 1.4% w/w,
- a pH adjuster, preferably in an amount between 0.1% w/w and 0.2% w/w, and/or a preservative agent, preferably in an amount between 0.1% w/w and 0.5% w/w.

In a preferred embodiment, the powder mixture according to the first object of the invention comprises:
- reparixin in an amount between 6.8% w/w and 28.2% w/w,
- a viscosity agent selected from xanthan gum in an amount between 0.3% w/w and 0.5% w/w, gum arabic in an amount between 0.3% w/w and 0.5% w/w, and Avicel CL-611 in an amount between 3% w/w and 8.3% w/w, preferably between 3% w/w and 8% w/w,
- sucrose in an amount between 63% w/w and 88% w/w,
- a wetting agent selected from polyvinylpyrrolidone in an amount between 3% w/w and 5% w/w and poloxamer 188 in an amount between 0.5% w/w and 1.5% w/w,
- colloidal silica in an amount between 0.7% w/w and 1.3% w/w,
with the proviso that when said viscosity agent is Avicel CL-611, said wetting agent is not polyvinylpyrrolidone.

In a preferred embodiment, the powder mixture according to the first object of the invention consists for at least 98%, preferably for at least 99% of its total weight, of:
- reparixin in an amount between 6.8% w/w and 28.2% w/w,
- a viscosity agent selected from xanthan gum in an amount between 0.3% w/w and 0.5% w/w, gum arabic in an amount between 0.3% w/w and 0.5% w/w, and Avicel CL-611 in an amount between 3% w/w and 8.3% w/w, preferably between 3% w/w and 8% w/w,
- sucrose in an amount between 63% w/w and 88% w/w,
- a wetting agent selected from polyvinylpyrrolidone in an amount between 3% w/w and 5% w/w, and poloxamer 188 in an amount between 0.5% w/w and 1.5% w/w,
- colloidal silica in an amount between 0.7% w/w and 1.3% w/w,
- a pH adjuster, preferably in an amount between 0.1 w/w and 0.2% w/w, and/or a preservative agent, preferably in an amount between 0.1% w/w and 0.5% w/w,
with the proviso that when said viscosity agent is Avicel CL-611, said wetting agent is not polyvinylpyrrolidone.

In a preferred embodiment, the powder mixture according to the first object of the invention comprises:
- reparixin in an amount between 6.8% w/w and 28.2% w/w,
- Avicel CL-611 in an amount between 3% w/w and 8.3% w/w, preferably between 3% w/w and 8% w/w,
- sucrose in an amount between 63% w/w and 88% w/w,
- a wetting agent selected from polyvinylpyrrolidone in an amount between 3% w/w and 5% w/w and poloxamer 188 in an amount between 0.5% w/w and 1.5% w/w,
- colloidal silica in an amount between 0.7% w/w and 1.3% w/w.

In a preferred embodiment, the powder mixture according to the first object of the invention consists for at least 98%, preferably for at least 99% of its total weight, of:
- reparixin in an amount between 6.8% w/w and 28.2% w/w,
- Avicel CL-611 in an amount between 3% w/w and 8.3% w/w, preferably between 3% w/w and 8% w/w,
- sucrose in an amount between 63% w/w and 88% w/w,
- poloxamer 188 in an amount between 0.5% w/w and 1.5% w/w,
- colloidal silica in an amount between 0.7% w/w and 1.3% w/w,
- a pH adjuster, preferably in an amount between 0.1 w/w and 0.2% w/w, and/or a preservative agent, preferably in an amount between 0.1% w/w and 0.5% w/w.

In a preferred embodiment, the powder mixture according to the first object of the invention comprises:
- reparixin in an amount between 7.6% w/w and 26.8% w/w,
- a viscosity agent selected from xanthan gum in an amount between 0.3% w/w and 0.5% w/w, gum arabic in an amount between 0.3% w/w and 0.5% w/w, and Avicel CL-611 in an amount between 4.5% w/w and 7.8% w/w,
- sucrose in an amount between 64.2% w/w and 85.2% w/w,
- a wetting agent selected from polyvinylpyrrolidone in an amount between 3% w/w and 5% w/w, and poloxamer 188 in an amount between 0.7% w/w and 1.3% w/w,
- colloidal silica in an amount between 0.7% w/w and 1.3% w/w,
with the proviso that when said viscosity agent is Avicel CL-611, said wetting agent is not polyvinylpyrrolidone.

In a preferred embodiment, the powder mixture according to the first object of the invention consists for at least 98%, preferably for at least 99% of its total weight, of:
- reparixin in an amount between 7.6% w/w and 26.8% w/w,
- a viscosity agent selected from xanthan gum in an amount between 0.3% w/w and 0.5% w/w, gum arabic in an amount between 0.3% w/w and 0.5% w/w and Avicel CL-611 in an amount between 4.5% w/w and 7.8% w/w,
- sucrose in an amount between 64.2% w/w and 85.2% w/w,
- a wetting agent selected from polyvinylpyrrolidone in an amount between 3% w/w and 5% w/w, and poloxamer 188 in an amount between 0.7% w/w and 1.3% w/w,
- colloidal silica in an amount between 0.7% w/w and 1.3% w/w
- a pH adjuster, preferably in an amount between 0.1% w/w and 0.2% w/w, and/or a preservative agent, preferably in an amount between 0.2% w/w and 0.4% w/w,
with the proviso that when said viscosity agent is Avicel CL-611, said wetting agent is not polyvinylpyrrolidone.

In a preferred embodiment, the powder mixture according to the first object of the invention comprises:
- reparixin in an amount between 7.6% w/w and 26.8% w/w,
- Avicel CL-611 in an amount between 4.5% w/w and 7.8% w/w,
- sucrose in an amount between 64.2% w/w and 85.2% w/w,
- poloxamer 188 in an amount between 0.7% w/w and 1.3% w/w,
- colloidal silica in an amount between 0.7% w/w and 1.3% w/w.

In a preferred embodiment, the powder mixture according to the first object of the invention consists for at least 98%, preferably for at least 99% of its total weight, of:
- reparixin in an amount between 7.6% w/w and 26.8% w/w,
- Avicel CL-611 in an amount between 4.5% w/w and 7.8% w/w,
- sucrose in an amount between 64.2% w/w and 85.2% w/w,
- poloxamer 188 in an amount between 0.7% w/w and 1.3% w/w,
- colloidal silica in an amount between 0.7% w/w and 1.3% w/w
- a pH adjuster, preferably in an amount between 0.1% w/w and 0.2% w/w, and/or a preservative agent, preferably in an amount between 0.2% w/w and 0.4% w/w.

In a preferred embodiment, the powder mixture according to the first object of the invention comprises:
- reparixin in an amount between 8.4% w/w and 26% w/w,
- a viscosity agent selected from xanthan gum in an amount between 0.3% w/w and 0.5% w/w, gum arabic in an amount between 0.3% w/w and 0.5% w/w, and Avicel CL-611 in an amount between 4.5% w/w and 7.7% w/w,
- sucrose in an amount between 64.9% w/w and 84.5% w/w,
- a wetting agent selected from polyvinylpyrrolidone in an amount between 3% w/w and 5% w/w, and poloxamer 188 in an amount between 0.7% w/w and 1.3% w/w,
- colloidal silica in an amount between 0.7% w/w and 1.3% w/w,
with the proviso that when said viscosity agent is Avicel CL-611, said wetting agent is not polyvinylpyrrolidone.

In a preferred embodiment, the powder mixture according to the first object of the invention consists for at least 98%, preferably for at least 99% of its total weight, of:
- reparixin in an amount between 8.4% w/w and 26% w/w,
- a viscosity agent selected from xanthan gum in an amount between 0.3% w/w and 0.5% w/w, gum arabic in an amount between 0.3% w/w and 0.5% w/w, and Avicel CL-611 in an amount between 4.5% w/w and 7.7% w/w,
- sucrose in an amount between 64.9% w/w and 84.5% w/w,
- a wetting agent selected from polyvinylpyrrolidone in an amount between 3% w/w and 5% w/w, and poloxamer 188 in an amount between 0.7% w/w and 1.3% w/w,
- colloidal silica in an amount between 0.7% w/w and 1.3% w/w
- a pH adjuster, preferably in an amount between 0.1% w/w and 0.2% w/w, and/or a preservative agent, preferably in an amount between 0.2% w/w and 0.4% w/w,
with the proviso that when said viscosity agent is Avicel CL-611, said wetting agent is not polyvinylpyrrolidone.

In a preferred embodiment, the powder mixture according to the first object of the invention comprises:
- reparixin in an amount between 8.4% w/w and 26% w/w,
- Avicel CL-611 in an amount between 4.5% w/w and 7.7% w/w,
- sucrose in an amount between 64.9% w/w and 84.5% w/w,
- poloxamer 188 in an amount between 0.7% w/w and 1.3% w/w,
- colloidal silica in an amount between 0.7% w/w and 1.3% w/w.

In a preferred embodiment, the powder mixture according to the first object of the invention consists for at least 98%, preferably for at least 99% of its total weight, of:
- reparixin in an amount between 8.4% w/w and 26% w/w,
- Avicel CL-611 in an amount between 4.5% w/w and 7.7% w/w,
- sucrose in an amount between 64.9% w/w and 84.5% w/w,
- poloxamer 188 in an amount between 0.7% w/w and 1.3% w/w,
- colloidal silica in an amount between 0.7% w/w and 1.3% w/w
- a pH adjuster, preferably in an amount between 0.1% w/w and 0.2% w/w, and/or a preservative agent, preferably in an amount between 0.2% w/w and 0.4% w/w.

In a preferred embodiment, the powder mixture according to the first object of the invention comprises:
- reparixin in an amount between 7.6% w/w and 13.9% w/w,
- sucrose in an amount between 75.5% w/w and 85.2% w/w,
- a viscosity agent selected from xanthan gum in an amount between 0.3% w/w and 0.5% w/w, gum arabic in an amount between 0.3% w/w and 0.5% w/w, and Avicel CL-611 in an amount between 5% w/w and 7.8% w/w,
- a wetting agent selected from polyvinylpyrrolidone in an amount between 3% w/w and 5% w/w, and poloxamer 188 in an amount between 0.8% w/w and 1.3% w/w,
- colloidal silica in an amout between 0.8% w/w and 1.3% w/w,
with the proviso that when said viscosity agent is Avicel CL-611, said wetting agent is not polyvinylpyrrolidone.

In a preferred embodiment, the powder mixture according to the first object of the invention consists for at least 98%, preferably for at least 99% of its total weight, of:
- reparixin in an amount between 7.6% w/w and 13.9% w/w,
- sucrose in an amount between 75.5% w/w and 85.2% w/w,
- a viscosity agent selected from xanthan gum in an amount between 0.3% w/w and 0.5% w/w, gum arabic in an amount between 0.3% w/w and 0.5% w/w, and Avicel CL-611 in an amount between 5% w/w and 7.8% w/w,
- a wetting agent selected from polyvinylpyrrolidone in an amount between 3% w/w and 5% w/w, and poloxamer 188 in an amount between 0.8% w/w and 1.3% w/w,
- a pH adjuster, preferably in an amount between 0.1% w/w and 0.17% w/w, and/or a preservative agent, preferably in an amount between 0.2% w/w and 0.4% w/w, wherein said pH adjuster is preferably monohydrate citric acid and/or said preservative agent is preferably potassium sorbate,
- colloidal silica in an amout between 0.8% w/w and 1.3% w/w,
with the proviso that when said viscosity agent is Avicel CL-611, said wetting agent is not polyvinylpyrrolidone.

In a preferred embodiment, the powder mixture according to the first object of the invention comprises:
- reparixin in an amount between 7.6% w/w and 13.9% w/w,
- sucrose in an amount between 75.5% w/w and 85.2% w/w,
- Avicel CL-611 in an amount between 5% w/w and 7.8% w/w,
- poloxamer 188 in an amount between 0.8% w/w and 1.3% w/w,
- colloidal silica in an amout between 0.8% w/w and 1.3% w/w.

In a preferred embodiment, the powder mixture according to the first object of the invention consists for at least 98%, preferably for at least 99% of its total weight, of:
- reparixin in an amount between 7.6% w/w and 13.9% w/w,
- sucrose in an amount between 75.5% w/w and 85.2% w/w,
- a preservative agent, preferably potassium sorbate, in an amount between 0.2% w/w and 0.4% w/w,
- Avicel CL-611 in an amount between 5% w/w and 7.8% w/w,
- poloxamer 188 in an amount between 0.8% w/w and 1.3% w/w,
- a pH adjuster, preferably monohydrate citric acid, in an amount between 0.1% w/w and 0.17% w/w,
- colloidal silica in an amout between 0.8% w/w and 1.3% w/w.

In a preferred embodiment, the powder mixture according to the first object of the invention comprises:
- reparixin in an amount between 8.4% w/w and 12.8% w/w,
- sucrose in an amount between 76.5% w/w and 84.5% w/w,
- a viscosity agent selected from xanthan gum in an amount between 0.3% w/w and 0.5% w/w, gum arabic in an amount between 0.3% w/w and 0.5% w/w, and Avicel CL-611 in an amount between 5% w/w and 7.7% w/w,
- a wetting agent selected from polyvinylpyrrolidone in an amount between 3% w/w and 5% w/w, and poloxamer 188 in an amount between 0.8% w/w and 1.3% w/w,
- colloidal silica in an amount between 0.8% w/w and 1.3% w/w,
with the proviso that when said viscosity agent is Avicel CL-611, said wetting agent is not polyvinylpyrrolidone.

In a preferred embodiment, the powder mixture according to the first object of the invention consists for at least 98%, preferably for at least 99% of its total weight, of:
- reparixin in an amount between 8.4% w/w and 12.8% w/w,
- sucrose in an amount between 76.5% w/w and 84.5% w/w,
- a viscosity agent selected from xanthan gum in an amount between 0.3% w/w and 0.5% w/w, gum arabic in an amount between 0.3% w/w and 0.5% w/w and Avicel CL-611 in an amount between 5% w/w and 7.7% w/w,
- a wetting agent selected from polyvinylpyrrolidone in an amount between 3% w/w and 5% w/w, and poloxamer 188 in an amount between 0.8% w/w and 1.3% w/w,
- a pH adjuster, preferably in an amount between 0.1% w/w and 0.2% w/w, and/or a preservative agent, preferably in an amount between 0.2% w/w and 0.4% w/w, wherein said pH adjuster is preferably monohydrate citric acid and/or said preservative agent is preferably potassium sorbate,
- colloidal silica in an amount between 0.8% w/w and 1.3% w/w,
with the proviso that when said viscosity agent is Avicel CL-611, said wetting agent is not polyvinylpyrrolidone.

In a preferred embodiment, the powder mixture according to the first object of the invention comprises:
- reparixin in an amount between 8.4% w/w and 12.8% w/w,
- sucrose in an amount between 76.5% w/w and 84.5% w/w,
- Avicel CL-611 in an amount between 5% w/w and 7.7% w/w,
- poloxamer 188 in an amount between 0.8% w/w and 1.3% w/w,
- colloidal silica in an amount between 0.8% w/w and 1.3% w/w.

In a preferred embodiment, the powder mixture according to the first object of the invention consists for at least 98%, preferably for at least 99% of its total weight, of:
- reparixin in an amount between 8.4% w/w and 12.8% w/w,
- sucrose in an amount between 76.5% w/w and 84.5% w/w,
- a preservative agent, preferably potassium sorbate, in an amount between 0.2% w/w and 0.4% w/w,
- Avicel CL-611 in an amount between 5% w/w and 7.7% w/w,
- poloxamer 188 in an amount between 0.8% w/w and 1.3% w/w,
- a pH adjuster, preferably monohydrate citric acid, in an amount between 0.1% w/w and 0.2% w/w,
- colloidal silica in an amount between 0.8% w/w and 1.3% w/w.

In another preferred embodiment, the powder mixture according to the first object of the invention comprises:
- reparixin in an amount between 17.5% w/w and 26.8% w/w,
- sucrose in an amount between 64.2% w/w and 76.1% w/w,
- a viscosity agent selected from xanthan gum in an amount between 0.3% w/w and 0.5% w/w, gum arabic in an amount between 0.3% w/w and 0.5% w/w and Avicel CL-611 in an amount between 4.5% w/w and 6.6% w/w,
- a wetting agent selected from polyvinylpyrrolidone in an amount between 3% w/w and 5% w/w, and poloxamer 188 in an amount between 0.7% w/w and 1.1% w/w,
- colloidal silica in an amount between 0.7% w/w and 1.1% w/w,
with the proviso that when said viscosity agent is Avicel CL-611, said wetting agent is not polyvinylpyrrolidone.

In another preferred embodiment, the powder mixture according to the first object of the invention consists for at least 98%, preferably for at least 99% of its total weight, of:
- reparixin in an amount between 17.5% w/w and 26.8% w/w
- sucrose in an amount between 64.2% w/w and 76.1% w/w
- a viscosity agent selected from xanthan gum in an amount between 0.3% w/w and 0.5% w/w, gum arabic in an amount between 0.3% w/w and 0.5% w/w and Avicel CL-611 in an amount between 4.5% w/w and 6.6% w/w,
- a wetting agent selected from polyvinylpyrrolidone in an amount between 3% w/w and 5% w/w, and poloxamer 188 in an amount between 0.7% w/w and 1.1% w/w,
- a pH adjuster, preferably in an amount between 0.1% w/w and 0.2% w/w, and/or a preservative agent, preferably in an amount between 0.2% w/w and 0.35% w/w, wherein said pH adjuster is preferably monohydrate citric acid and/or said preservative agent is preferably potassium sorbate,
- colloidal silica in an amount between 0.7% w/w and 1.1% w/w,
with the proviso that when said viscosity agent is Avicel CL-611, said wetting agent is not polyvinylpyrrolidone.

In another preferred embodiment, the powder mixture according to the first object of the invention comprises:
- reparixin in an amount between 17.5% w/w and 26.8% w/w,
- sucrose in an amount between 64.2% w/w and 76.1% w/w,
- Avicel CL-611 in an amount between 4.5% w/w and 6.6% w/w,
- poloxamer 188 in an amount between 0.7% w/w and 1.1% w/w,
- colloidal silica in an amount between 0.7% w/w and 1.1% w/w.

In another preferred embodiment, the powder mixture according to the first object of the invention consists for at least 98%, preferably for at least 99% of its total weight, of:
- reparixin in an amount between 17.5% w/w and 26.8% w/w
- sucrose in an amount between 64.2% w/w and 76.1% w/w
- a preservative agent, preferably potassium sorbate, in an amount between 0.2% w/w and 0.35% w/w,
- Avicel CL-611 in an amount between 4.5% w/w and 6.6% w/w,
- poloxamer 188 in an amount between 0.7% w/w and 1.1% w/w,
- a pH adjuster, preferably monohydrate citric acid, in an amount between 0.1% w/w and 0.2% w/w
- colloidal silica in an amount between 0.7% w/w and 1.1% w/w.

In another preferred embodiment, the powder mixture according to the first object of the invention comprises:
- reparixin in an amount between 18.1% w/w and 26% w/w,
- sucrose in an amount between 64.9% w/w and 75.6% w/w,
- a viscosity agent selected from xanthan gum in an amount between 0.3% w/w and 0.5% w/w, gum arabic in an amount between 0.3% w/w and 0.5% w/w and Avicel CL-611 in an amount between 4.5% w/w and 6.5% w/w,
- a wetting agent selected from polyvinylpyrrolidone in an amount between 3% w/w and 5% w/w, and poloxamer 188 in an amount between 0.75% w/w and 1.1% w/w,
- colloidal silica in an amount between 0.7% w/w and 1.1% w/w,
with the proviso that when said viscosity agent is Avicel CL-611, said wetting agent is not polyvinylpyrrolidone.

In another preferred embodiment, the powder mixture according to the first object of the invention consists for at least 98%, preferably for at least 99% of its total weight, of:
- reparixin in an amount between 18.1% w/w and 26% w/w,
- sucrose in an amount between 64.9% w/w and 75.6% w/w,
- a viscosity agent selected from xanthan gum in an amount between 0.3% w/w and 0.5% w/w, gum arabic in an amount between 0.3% w/w and 0.5% w/w and Avicel CL-611 in an amount between 4.5% w/w and 6.5% w/w,
- a wetting agent selected from polyvinylpyrrolidone in an amount between 3% w/w and 5% w/w, and poloxamer 188 in an amount between 0.75% w/w and 1.1% w/w,
- a pH adjuster, preferably in an amount between 0.1% w/w and 0.15% w/w, and/or a preservative agent, preferably in an amount between 0.2% w/w and 0.35% w/w, wherein said pH adjuster is preferably monohydrate citric acid and/or said preservative agent is preferably potassium sorbate,
- colloidal silica in an amount between 0.7% w/w and 1.1% w/w,
with the proviso that when said viscosity agent is Avicel CL-611, said wetting agent is not polyvinylpyrrolidone.

In another preferred embodiment, the powder mixture according to the first object of the invention comprises:
- reparixin in an amount between 18.1% w/w and 26% w/w,
- sucrose in an amount between 64.9% w/w and 75.6% w/w,
- Avicel CL-611 in an amount between 4.5% w/w and 6.5% w/w,
- poloxamer 188 in an amount between 0.75% w/w and 1.1% w/w,
- colloidal silica in an amount between 0.7% w/w and 1.1% w/w.

In another preferred embodiment, the powder mixture according to the first object of the invention consists for at least 98%, preferably for at least 99% of its total weight, of:
- reparixin in an amount between 18.1% w/w and 26% w/w,
- sucrose in an amount between 64.9% w/w and 75.6% w/w,
- a preservative agent, preferably potassium sorbate, in an amount between 0.2% w/w and 0.35% w/w,
- Avicel CL-611 in an amount between 4.5% w/w and 6.5% w/w,
- poloxamer 188 in an amount between 0.75% w/w and 1.1% w/w,
- a pH adjuster, preferably monohydrate citric acid, in an amount between 0.1% w/w and 0.15% w/w,
- colloidal silica in an amount between 0.7% w/w and 1.1% w/w.

Preferably, when polyvinylpyrrolidone is used as wetting agent, said polyvinylpyrrolidone is polyvinylpyrrolidone K 30.

In an embodiment, also in combination with any of the above-described embodiments, said pH adjuster is able to adjust the pH of the liquid suspension reconstituted from the powder mixture at a value between 4 and 6, preferably between 4.5 and 5.5, more preferably 5.

In an embodiment, also in combination with any of the above-described embodiments, said pH adjuster is selected from citric acid, preferably monohydrate citric acid, hydrochloric acid, sodium phosphate, preferably sodium phosphate monobasic, potassium phosphate, preferably potassium phosphate monobasic.

In an embodiment, also in combination with any of the above-described embodiments, said pH adjuster is citric acid, preferably monohydrate citric acid, in an amount between 0.1% w/w and 0.2% w/w.

In an embodiment, also in combination with any of the above-described embodiments, said preservative agent is selected from potassium sorbate, sodium benzoate, methyl parabens and propyl parabens. In a preferred embodiment, also in combination with any of the above-described embodiments, said preservative agent is potassium sorbate in an amount between 0.1% w/w and 0.5% w/w.

Other preferred embodiments of the powder mixture according to the first object of the invention are shown in Table 1, Table 2, Table 3 and Table 4 below. All the values in the Tables are to be interpreted as % w/w as defined above.

**Table 1**

| | | | | | | | | | | |
|---|---|---|---|---|---|---|---|---|---|---|
| **Reparixin** | 12.75 | 12.44 | 12.13 | 11.85 | 11.57 | 11.31 | 11.06 | 10.82 | 10.59 | 10.37 |
| **Sucrose** | 76.52 | 77.11 | 77.66 | 78.19 | 78.70 | 79.18 | 79.64 | 80.08 | 80.50 | 80.91 |
| **Potassium sorbate** | 0.38 | 0.37 | 0.36 | 0.36 | 0.35 | 0.34 | 0.33 | 0.32 | 0.32 | 0.31 |
| **Avicel CL-611** | 7.65 | 7.46 | 7.28 | 7.11 | 6.94 | 6.79 | 6.64 | 6.49 | 6.36 | 6.22 |
| **Poloxamer 188** | 1.28 | 1.24 | 1.21 | 1.18 | 1.16 | 1.13 | 1.11 | 1.08 | 1.06 | 1.04 |
| **Monohydrate cytric acid** | 0.16 | 0.16 | 0.16 | 0.15 | 0.15 | 0.14 | 0.14 | 0.14 | 0.14 | 0.13 |
| **Colloidal silica** | 1.25 | 1.22 | 1.19 | 1.16 | 1.13 | 1.11 | 1.08 | 1.06 | 1.04 | 1.02 |

**Table 2**

| | | | | | | | | | | | |
|---|---|---|---|---|---|---|---|---|---|---|---|
| **Reparixin** | 10.16 | 9.96 | 9.76 | 9.58 | 9.40 | 9.22 | 9.06 | 8.90 | 8.74 | 8.59 | 8.45 |
| **Sucrose** | 81.29 | 81.67 | 82.02 | 82.37 | 82.70 | 83.02 | 83.33 | 83.62 | 83.91 | 84.19 | 84.45 |
| **Potassium sorbate** | 0.30 | 0.30 | 0.29 | 0.29 | 0.28 | 0.28 | 0.27 | 0.27 | 0.26 | 0.26 | 0.25 |
| **Avicel CL-611** | 6.10 | 5.98 | 5.86 | 5.75 | 5.64 | 5.53 | 5.43 | 5.34 | 5.24 | 5.15 | 5.07 |
| **Poloxamer 188** | 1.02 | 1.00 | 0.98 | 0.96 | 0.94 | 0.92 | 0.91 | 0.89 | 0.87 | 0.86 | 0.84 |
| **Monohydrate cytric acid** | 0.13 | 0.13 | 0.12 | 0.12 | 0.12 | 0.12 | 0.12 | 0.11 | 0.11 | 0.11 | 0.11 |
| **Colloidal silica** | 1.00 | 0.98 | 0.96 | 0.94 | 0.92 | 0.90 | 0.89 | 0.87 | 0.86 | 0.84 | 0.83 |

**Table 3**

| | | | | | | | | | | |
|---|---|---|---|---|---|---|---|---|---|---|
| **Reparixin** | 25.97 | 25.42 | 24.89 | 24.39 | 23.90 | 23.44 | 22.99 | 22.55 | 22.14 | 21.74 |
| **Sucrose** | 64.93 | 65.67 | 66.38 | 67.07 | 67.72 | 68.35 | 68.96 | 69.54 | 70.11 | 70.65 |
| **Potassium sorbate** | 0.32 | 0.32 | 0.31 | 0.30 | 0.30 | 0.29 | 0.29 | 0.28 | 0.28 | 0.27 |
| **Avicel - CL611** | 6.49 | 6.36 | 6.22 | 6.10 | 5.98 | 5.86 | 5.75 | 5.64 | 5.53 | 5.43 |
| **Poloxamer 188** | 1.08 | 1.06 | 1.04 | 1.02 | 1.00 | 0.98 | 0.96 | 0.94 | 0.92 | 0.91 |
| **Monohydrate cytric acid** | 0.14 | 0.14 | 0.13 | 0.13 | 0.13 | 0.12 | 0.12 | 0.12 | 0.12 | 0.12 |
| **Colloidal silica** | 1.06 | 1.04 | 1.02 | 1.00 | 0.98 | 0.96 | 0.94 | 0.92 | 0.90 | 0.89 |

**Table 4**

| | | | | | | | | | | | |
|---|---|---|---|---|---|---|---|---|---|---|---|
| **Reparixin** | 21.35 | 20.98 | 20.62 | 20.27 | 19.93 | 19.61 | 19.29 | 18.99 | 18.69 | 18.40 | 18.13 |
| **Sucrose** | 71.17 | 71.67 | 72.16 | 72.63 | 73.08 | 73.52 | 73.95 | 74.36 | 74.76 | 75.15 | 75.52 |
| **Potassium sorbate** | 0.27 | 0.26 | 0.26 | 0.25 | 0.25 | 0.25 | 0.24 | 0.24 | 0.23 | 0.23 | 0.23 |
| **Avicel CL-611** | 5.34 | 5.24 | 5.15 | 5.07 | 4.98 | 4.90 | 4.82 | 4.75 | 4.67 | 4.60 | 4.53 |
| **Poloxamer 188** | 0.89 | 0.87 | 0.86 | 0.84 | 0.83 | 0.82 | 0.80 | 0.79 | 0.78 | 0.77 | 0.76 |
| **Monohydrate cytric acid** | 0.11 | 0.11 | 0.11 | 0.11 | 0.11 | 0.10 | 0.10 | 0.10 | 0.10 | 0.10 | 0.10 |
| **Colloidal silica** | 0.87 | 0.86 | 0.84 | 0.83 | 0.81 | 0.80 | 0.79 | 0.78 | 0.76 | 0.75 | 0.74 |

In an embodiment, also in combination with any of the above embodiments, the powder mixture according to the first object of the invention has a flowability index between 10 mm and 4 mm, preferably of 6 mm, wherein said flowability index is measured according to the procedure described in Example 2 below by using Flodex Apparatus, Teledyne Hanson Research, Chatsworth, California, USA.

A second object of the present invention is a process to produce a liquid suspension comprising reparixin including the step of suspending the powder mixture according to the first object of the invention in an aqueous vehicle, preferably water. The amount of water in which reparixin is suspended may vary based on the amount of reparixin, and the skilled person is able to determine such amount of water.

In an embodiment, the liquid suspension reconstituted from the powder mixture has a zeta potential value between -5 mV and +5mV, preferably between -3 mV and +3 mV, wherein said zeta potential value is measured by laser Doppler electrophoresis using Zetasizer nano ZS (Malvern Instruments, Worcestershire, UK) according to the manufacturer's instructions.

In an embodiment, also in combination with any of the above-described embodiments, the reconstituted liquid suspension has a yield stress value of at least 1.3 Pa, wherein said yield stress value is measured by Rheometer MCR 102 (Anton Paar, Graz, Austria) following the procedure described in Example 2 below.

A third object of the invention is the powder mixture according to any one of the above-described embodiments or the reconsituted liquid suspension thereof for use in the treatment of a disease or condition selected from psoriasis, rheumatoid arthritis, ulcerative colitis, acute respiratory distress syndrome (ARDS), idiopathic fibrosis and glomerulonephritis.

A further object of the invention is the powder mixture according to any one of the above-described embodiments or the reconsituted liquid suspension thereof for use in the prevention of diabetes or in delaying the onset and the progression of diabetes, wherein said diabetes is preferably Type I diabetes.

A further object the present invention is the powder mixture according to any one of the above-described embodiments or the reconstituted liquid suspension thereof for use in the treatment of an infection caused by SARS-CoV-2 or a variant thereof, preferably for use in the treatment of COVID-19, even more preferably for use in the improvement of respiratory function in subjects affected by pneumonia caused by SARS-CoV-2 or a variant thereof.

In an embodiment, said improvement of respiratory function in said subjects means decreasing, delaying the onset of or preventing the need of supplemental oxygen requirement, mechanical ventilation use or admission to Intensive Care Unit.

As used herein, the term "pneumonia caused by SARS-CoV-2 or a variant thereof" indicates an inflammatory condition of the lung associated to an infection with SARS-CoV-2 or a variant thereof. This is a severe complication of COVID-19 affecting a percentage of the subjects.

In an embodiment, the liquid suspension according to any one of the above-described embodiments for use in the treatment of COVID-19 is administered in combination with a standard of care treatment for COVID-19 patients.

As used herein, by "standard of care treatment for COVID-19 patients" it is meant a pharmacological treatment that includes one or more drugs that have been approved by regulatory authorities i) as a treatment for COVID-19 or ii) as a treatment for other pathological conditions and symptoms thereof and that are used in clinical practice as a treatment of symptoms and complications that are associated to COVID-19. Preferably, said standard of care treatment consists in the treatment of the patient with at least one drug selected from: i) antiviral drugs, preferably remdesivir; ii) antipyretic, analgesic and antiinflammatory drugs, preferably non-steroidal anti inflammatory drugs, corticosteroids, cytokine, chemokine and interleukin inhibitors, more preferably selected from dexhamethasone, paracetamol, anakinra, celecoxib, prednisone, prednisolone, methylprednisolone, piryrone, and tramadol; iii) antibiotics, preferably selected from piperacillin in association with tazobactam, azithromycin and ceftriazone; and iv) anticoagulants and antithrombotic drugs, preferably selected from enoxaparin and acetylsalicylic acid.

Preferably, said subjects affected by pneumonia caused by SARS-CoV-2 or a variant thereof have lymphocytopenia.

According to a preferred embodiment, said subjects with lymphocytopenia are adult patients (14 years and above) and have blood concentration of lymphocytes below 1000, below 900, below 850, below 800 or below 750 cells/microliter.

According to another preferred embodiment, said patients with lymphocytopenia are pediatric patients (below 14 years of age) and have blood concentration of lymphocytes below 3000, below 2800, below 2500, below 2300 or below 2000 cells/microliter.

A further object of the present invention is the powder mixture according to any one of the above-described embodiments or the reconsituted liquid suspension thereof for use in the prevention or treatment of seizures in an individual.

According to a preferred embodiment, said seizures are acute symptomatic seizures (ASS).

According to another preferred embodiment, said seizures are associated to an established epilepsy (Established Epilepsy Seizures EES).

In an embodiment, the reconstituted liquid suspension for use according to the present invention is administered orally to a subject, preferably a mammal, even more preferably a human.

In an embodiment, the powder mixture according to the first object of the invention comprising reparixin in an amount between 7.6% w/w and 13.9% w/w and/or the reconstituted liquid suspension thereof is suitable for administration to a pediatric subject, wherein the pediatric subject is less than 14 years of life.

In an embodiment, the powder mixture according to the first object of the invention comprising reparixin in an amount between 17.5% w/w and 26.8% w/w and/or the reconstituted liquid suspension thereof is suitable for administration to an adult subject, wherein the adult subject is more than 14 years of life.

A further object of the present invention is a kit comprising, preferably consisting of:
a) a powder mixture according to any of the above-described embodiments of the first object of the invention, and
b) an aqueous vehicle, preferably water.

In an embodiment, a) and b) are contained in separated containers. In an alternative embodiment, a) and b) are contained in the same container and separated by a breakable barrier, which can be disrupted at the time of use to disperse the powder mixture in the aqueous vehicle.

The invention will be further described in the following examples, which do not limit the scope of the invention as defined in the claims.

### EXPERIMENTAL SECTION

All the percentages indicated in the following tables are to be intended as %w/w.

### Example 1 - Preparation of liquid suspensions and powder mixtures comprising reparixin

The following experimental procedure was followed:
a) liquid suspensions comprising reparixin were prepared and characterized in terms of zeta potential and rheological properties;
b) based on the results of the characterization, the best suspensions were selected and the corresponding powder mixtures were prepared.

The materials used for preparing the suspensions are indicated in Table 5.

**Table 5**

| **Trade Name** | **Chemical Name** | **Supplier** |
|---|---|---|
| Sucrose RFF | Sucrose | Südzucker |
| Sucralose | Sucralose | Merck-Millipore |
| Potassium Sorbate | Potassium Sorbate | Merck-Millipore |
| Avicel CL-611 | Sodium Carboxymethylcellulose + microcrystalline cellulose | Dupont |
| Xantural 75 | Xanthan Gum | Kelco |
| SLS | Sodium Lauryl Sulphate | Basf |
| Kolliphor P188 Micro (Poloxamer 188) | polyoxyethylene-polyoxypropylene copolymer average MW 7608-9510 | Basf |
| Kollidon 30 | Polyvinylpyrrolidone | Basf |
| Kolliphor P407 (Poloxamer 407) | polyoxyethylene-polyoxypropylene copolymer average MW 9840-14600 | Basf |
| Simethicone | methoxy-dimethyl-trimethylsilyloxysilane | Wacker |
| Monohydrate Citric Acid | Monohydrate Citric Acid | Merck Millipore |
| Dihydrate Sodium Citrate | Dihydrate Sodium Citrate | Sigma-Aldrich |
| Kollidon CL-M (crospovidone) | 1-Ethenyl-2pyrrolidinone homopolymer | Basf |
| Aeroperl 330 Pharma | Colloidal Silica | Evonik |

The prepared suspensions contained the following excipients:
- a wetting agent, which is used to increase the wettability of reparixin in water, which is per se low;
- a sweetener, which is used to mask the unpleasant taste of reparixin;
- a preservative agent, which is used for preventing or retarding microbial growth;
- a pH adjuster, which is used for regulating the pH of the solution at a desired value.

The type and/or amount of the following components were varied among the suspensions:
- the wetting agent; in particular, sodium lauryl sulfate (SLS), poloxamer 407, polyvinylpyrrolidone and poloxamer 188 were tested as wetting agents. Poloxamer 407 was immediately rejected due to its insufficient ability to disperse reparixin;
- the sweetener; in particular, sucrose and sucralose were tested as sweeteners. Sucrose has a high glycemic index, while sucralose is an artificial sweetener with zero glycemic index.

The procedure used for the preparation of the liquid suspensions is described in the following.

The selected amounts of antifoaming agent (when present), wetting agent, preservative agent and pH adjuster(s) were added to 40 g of distilled water, and the mixture was maintained under magnetic stirrer until a solution was obtained. Then, the selected amount of reparixin was added and dispersed. Once the API was homogenously suspended, the selected amounts of other excipients (viscosity agent and sweetener) were added and dissolved. Finally, water was added up to a final weight of 100 g.

In the following, the composition of the prepared liquid suspensions and powder mixtures will be reported, while the respective data of characterization will be shown and discussed in Example 2 below.

Table 6 below reports the liquid suspensions prepared by using SLS as wetting agent. Specifically, the prepared suspensions contained 0.11% w/w SLS, 40% w/w sucrose as sweetener, 0.15% w/w potassium sorbate as preservative agent, monohydrate citric acid and dihydrate sodium citrate as pH adjusters, 0.005% w/w simethicone as antifoaming agent and water as solvent.

The amounts of pH adjusters (monohydrate citric acid and dihydrate sodium citrate) and the type/amount of viscosity agent (xanthan gum or Avicel CL-611) were varied among the different suspensions prepared.

**Table 6**

| **Suspension** | **41** | **55** | **56** | **57** | **58** | **59** |
|---|---|---|---|---|---|---|
| **Reparixin** | 5.00% | 5.00% | 5.00% | 5.00% | 5.00% | 5.00% |
| **Sucrose** | 40.00% | 40.00% | 40.00% | 40.00% | 40.00% | 40.00% |
| **Potassium sorbate** | 0.15% | 0.15% | 0.15% | 0.15% | 0.15% | 0.15% |
| **Viscosity agent** | Avicel CL-611 1.00% | Xanthan gum 0.10% | Xanthan gum 0.25% | Avicel CL-611 1.00% | Avicel CL-611 1.00% | Avicel CL-611 1.00% |
| **Monohydrate citric acid** | 0.064% | 0.064% | 0.064% | 0.320% | 0.640% | 1.240% |
| **Dihydrate sodium citrate** | 0.208% | 0.208% | 0.208% | 1.040% | 2.080% | 4.140% |
| **Simethicone** | 0.005% | 0.005% | 0.005% | 0.005% | 0.005% | 0.005% |
| **SLS** | 0.11% | 0.11% | 0.11% | 0.11% | 0.11% | 0.11% |
| **Water** | Up to 100% | Up to 100% | Up to 100% | Up to 100% | Up to 100% | Up to 100% |

Table 7 below reports the liquid suspensions prepared by using poloxamer 188 as wetting agent. Specifically, the prepared suspensions contained 0.25% w/w poloxamer 188, Avicel CL-611 as viscosity agent, 0.15% w/w potassium sorbate as preservative agent, 40% w/w sucrose as sweetener, 0.005% w/w simethicone as antifoaming agent and water as solvent.

The amounts of Avicel CL-611 and the type/amount of pH adjusters (monohydrate citric acid/dihydrate sodium citrate or monohydrate citric acid/NaOH 1M) were varied among the different suspensions prepared.

In addition to the suspensions reported in Table 7, also suspension **80,** having a composition corresponding to suspension **79** but having 1.00% w/w Blanose (sodium carboxymethylcellulose) instead of 3.00% w/w Avicel CL-611, was prepared.

**Table 7**

| **Suspension** | **68** | **69** | **70** | **71** | **72** | **78** | **79** |
|---|---|---|---|---|---|---|---|
| **Reparixin** | 5.00% | 5.00% | 5.00% | 5.00% | 5.00% | 5.00% | 5.00% |
| **Sucrose** | 40.00% | 40.00% | 40.00% | 40.00% | 40.00% | 40.00% | 40.00% |
| **Potassium sorbate** | 0.15% | 0.15% | 0.15% | 0.15% | 0.15% | 0.15% | 0.15% |
| **Avicel CL-611** | 1.00% | 1.00% | 1.00% | 1.00% | 1.00% | 2.00% | 3.00% |
| **Monohydrate citric acid** | 0.320% | 0.10% | 0.40% | 0.40% | 0.40% | 0.064% | 0.064% |
| **Other buffering agent** | Dihydrate sodium citrate 1.040% | NaOH 1M 0.03% | NaOH 1M 0.06% | NaOH 1M 0.126% | NaOH 1M 0.162% | Dihydrate sodium citrate 0.208% | Dihydrate sodium citrate 0.208% |
| **Simethicone** | 0.005% | 0.005% | 0.005% | 0.005% | 0.005% | 0.005% | 0.005% |
| **Poloxamer 188** | 0.25% | 0.25% | 0.25% | 0.25% | 0.25% | 0.25% | 0.25% |
| **Water** | Up to 100% | Up to 100% | Up to 100% | Up to 100% | Up to 100% | Up to 100% | Up to 100% |

Table 8 below reports the liquid suspensions prepared by using polyvinylpyrrolidone as wetting agent and Avicel CL-611 as viscosity agent. Specifically, the prepared suspensions contained 2.00% w/w polyvinylpyrrolidone (PVP) K30, Avicel CL-611, 0.15% w/w potassium sorbate as preservative agent, 40% w/w sucrose as sweetener, 0.005% w/w simethicone as antifoaming agent, 0.064% w/w monohydrate citric acid and 0.208% w/w dihydrate sodium citrate as pH adjusters, and water as solvent.

The amounts of Avicel CL-611 were varied among the suspensions. Furthermore, suspension **104** also contained 1.00% w/w crospovidone, used as anticaking agent.

**Table 8**

| **Suspension** | **99** | **104** | **108** |
|---|---|---|---|
| **Reparixin** | 5.00% | 5.00% | 5.00% |
| **Sucrose** | 40.00% | 40.00% | 40.00% |
| **Potassium sorbate** | 0.15% | 0.15% | 0.15% |
| **Avicel CL-611** | 1.00% | 1.00% | 2.00% |
| **Monohydrate citric acid** | 0.064% | 0.064% | 0.064% |
| **Dihydrate sodium citrate** | 0.208% | 0.208% | 0.208% |
| **Simethicone** | 0.005% | 0.005% | 0.005% |
| **PVP K30** | 2.00% | 2.00% | 2.00% |
| **Crospovidone** | / | 1.00% | / |
| **Water** | Up to 100% | Up to 100% | Up to 100% |

Table 9 below reports other liquid suspensions prepared by using poloxamer 188 as wetting agent and Avicel CL-611 as viscosity agent. With respect to the suspensions reported in Table 7 above, both the amounts of poloxamer 188 and of Avicel CL-611 were increased. Specifically, the prepared suspensions contained 0.50% w/w Poloxamer 188, Avicel CL-611, 0.15% w/w potassium sorbate as preservative agent, 40.00% w/w sucrose as sweetener, 0.064% w/w monohydrate citric acid 0.208% w/w dihydrate sodium citrate as pH adjusters, and water as solvent.

The amounts of Avicel CL-611 and the amounts of simethicone, used in some suspensions as antifoaming agent, and the amounts of dihydrate sodium citrate, used in combination with monohydrate citric acid as pH adjuster (0.208% w/w or 0%) were varied among the suspensions prepared.

**Table 9**

| **Suspension** | **113** | **120** | **131** | **133** |
|---|---|---|---|---|
| **Reparixin** | 5% | 5% | 5% | 5% |
| **Sucrose** | 40.00% | 40.00% | 40.00% | 40.00% |
| **Potassium Sorbate** | 0.15% | 0.15% | 0.15% | 0.15% |
| **Avicel CL-611** | 2.00% | 2.50% | 3.00% | 3.00% |
| **Poloxamer P188** | 0.50% | 0.50% | 0.50% | 0.50% |
| **Monohydrate cytric acid** | 0.064% | 0.064% | 0.064% | 0.064% |
| **Dihydrate sodium citrate** | 0.208% | 0.208% | / | / |
| **Simethicone** | 0.005% | 0.005% | 0.005% | / |
| **Water** | Up to 100% | Up to 100% | Up to 100% | Up to 100% |

Table 10 below reports the powder mixtures prepared through geometric dilution using a powder mixer Turbula T2 F (WAB Group, Muttenz, Switzerland) according to manufacturer's instructions.

Powder mixtures, in amounts able to result in the desired amount of reparixin and of other components in the corresponding reconstituted liquid suspensions, were aliquoted into glass bottles and then re-constituted with water until a final volume of 100 g was reached. In some powder mixtures, different amounts of colloidal silica were introduced to investigate its effect on the flowability of the powder mixtures.

**Table 10**

| **Formulation** | **138** | **141** | **144** | **145** |
|---|---|---|---|---|
| **Reparixin** | 10.26% | 10.16% | 10.21% | 10.19% |
| **Sucrose** | 82.11% | 81.29% | 81.70% | 81.50% |
| **Potassium Sorbate** | 0.31% | 0.30% | 0.31% | 0.31% |
| **Avicel CL-611** | 6.16% | 6.10% | 6.13% | 6.11% |
| **Poloxamer P188** | 1.03% | 1.02% | 1.02% | 1.02% |
| **Monohydrate cytric acid** | 0.13% | 0.13% | 0.13% | 0.13% |
| **Colloidal silica** | / | 1.00% | 0.50% | 0.75% |

Table 11 below reports further powder mixtures prepared varying the percentage of reparixin and of sucrose. The powder mixture named "**141 pd**" in Table 11 corresponds to the powder mixture named "**141**" in Table 10. The corresponding reconstituted liquid suspensions are shown in Table 12. The abbreviation "**pd**" is used to indicate "powder mixtures", while the abbreviation "**lq**" is used to indicate "liquid".

**Table 11**

| **Formulation** | **163 pd** | **161 pd** | **141 pd** | **162 pd** | **164 pd** | **167 pd** | **150 pd** |
|---|---|---|---|---|---|---|---|
| **Reparixin** | 17.12% | 12.75% | 10.16% | 8.45% | 7.23% | 51.00% | 21.35% |
| **Sweetener** | Sucrose 68.48% | Sucrose 76.52% | Sucrose 81.29% | Sucrose 84.45% | Sucrose 86.70% | Sucralose 6.12% | Sucrose 71.17% |
| **Potassium sorbate** | 0.51% | 0.38% | 0.30% | 0.25% | 0.22% | 1.53% | 0.27% |
| **Avicel CL-611** | 10.27% | 7.65% | 6.10% | 5.07% | 4.34% | 30.60% | 5.34% |
| **Poloxamer 188** | 1.71% | 1.28% | 1.02% | 0.84% | 0.72% | 5.10% | 0.89% |
| **Monohydrate citric acid** | 0.22% | 0.16% | 0.13% | 0.11% | 0.09% | 0.65% | 0.11% |
| **Colloidal silica** | 1.68% | 1.25% | 1.00% | 0.83% | 0.71% | 5.00% | 0.87% |

**Table 12**

| **Suspension** | **163 lq** | **161 lq** | **141 lq** | **162 lq** | **164 lq** | **167 lq** | **150 lq** |
|---|---|---|---|---|---|---|---|
| **Reparixin** | 5.00% | 5.00% | 5.00% | 5.00% | 5.00% | 5.00% | 12.00% |
| **Sweetener** | Sucrose 20.00% | Sucrose 30.00% | Sucrose 40.00% | Sucrose 50.00% | Sucrose 60.00% | Sucralose 0.60% | Sucrose 40.00% |
| **Potassium sorbate** | 0.15% | 0.15% | 0.15% | 0.15% | 0.15% | 0.15% | 0.15% |
| **Avicel CL-611** | 3.00% | 3.00% | 3.00% | 3.00% | 3.00% | 3.00% | 3.00% |
| **Poloxamer 188** | 0.50% | 0.50% | 0.50% | 0.50% | 0.50% | 0.50% | 0.50% |
| **Monohydrate citric acid** | 0.064% | 0.064% | 0.064% | 0.064% | 0.064% | 0.064% | 0.064% |
| **Colloidal silica** | 0.49% | 0.49% | 0.49% | 0.49% | 0.49% | 0.49% | 0.49% |
| **Water** | Up to 100% | Up to 100% | Up to 100% | Up to 100% | Up to 100% | Up to 100% | Up to 100% |

### Example 2 - Characterization of the liquid suspensions and powder mixtures produced

The zeta potential values and the rheological properties of the liquid suspensions reported in Example 1 were determined. In case of powder mixtures, the flowability was evaluated. Reparixin is an insoluble active pharmaceutical ingredient that has large particle size and produces coarse suspensions. Because of this large particle size, the level of stability of suspensions containing reparixin is determined by gravitational forces, rather than by electrostatic interactions, which are insufficient to provide stability and kinetic stabilization. To ensure stability of the suspension, the zeta potential value has to be equal or near zero, and the particles of the internal phase must be surrounded by a hydration layer which hides their charge and ensures that, even when a sediment is formed, it is a loose sediment which is easily resuspendable, instead of a compact sediment difficult to resuspend. The increase of zeta potential (in terms of absolute value) corresponds to a condition where this hydration layer surrounding the particles of the internal phase is less present or absent, leading to an increase of the sedimentation rate. As regards the rheological properties, yield stress and thixotropy of the prepared suspensions were determined to gain information about the physical stability of the suspensions. The yield stress (τ0) is a material property that can be defined as the stress corresponding to the yield point at which the material begins to flow or deform plastically. A suspension is considered stable if the yield stress is higher than the sedimentation stress (τS), which can be defined as the stress a particle performs on the liquid. If τ0 is higher than τS, sedimentation will not occur, and the suspension can be considered stable. Generally, a yield stress around 1.3 Pa or more is considered a satisfactory value against sedimentation for liquid suspensions reconstituted at the time of use by suspending powder mixtures in an appropriate vehicle.

The physical stability of a suspension is also evaluated based on the thixotropy hysteresis area. Thixotropy is an imperative property of the polymer system selected as viscosity agent. Indeed, the consistency (e.g. viscosity) of the external phase, caused by the suspending agent, should decrease when a shear stress, such as the manual shaking, is applied so as to guarantee the re-dispersibility of sediment. In literature, the thixotropy area is usually correlated with the suspension stability. As demonstrated by E. C. Foernzler, the sedimentation velocity is directly proportional to the reciprocal of the hysteresis area, which means that the physical stability is directly proportional to thixotropy (Ernest C. Foernzler et al., J. Am. Pharm. Assoc. 49 (1960) 249-252).

Based on the above, the produced suspensions were characterized in terms of zeta potential value, rheological properties and capability of re-dispersion.

The values of zeta potential of the prepared suspensions were measured by laser Doppler electrophoresis using Zetasizer nano ZS (Malvern Instruments, Worcestershire, UK) according to manufacturer's instructions.

Rheological properties such as yield stress and thixotropy (hysteresis) were evaluated through Rheometer MCR 102 (Anton Paar, Graz, Austria).

The determination of yield stress was performed increasing linearly the shear rate stepwise between 0.1 and 100 s⁻¹ to generate a flow curve (shear stress vs. shear rate). Then, the resulting flow curve was fitted with a Herschel-Bulkley regression model, and the yield point was determined by extrapolating the flow curve to zero shear rate.

The thixotropy of the suspensions was evaluated via hysteresis. This measurement was carried out using an initial pre-shear at 5 1/s, the up-flow curve was then generated increasing the shear rate from 5 to 131 s⁻¹ (log scale) and the down-flow curve was obtained decreasing the shear rate from 131 to 5 s⁻¹. The area between the up-curve and down-curve was calculated and it is correlated with the thixotropy of the system. All analyses were carried out at room temperature using a 25 mm measuring plate and setting-up the gap at 1 mm.

If sediment was present, the capability of re-dispersion was evaluted considering the number of 180° rotations allowing the re-dispersion of sediment.

Table 13 below reports the values of zeta potential and rheological properties obtained for the suspensions reported in Table 6 above.

**Table 13**

| **Suspension** | **Zeta Potential (mV)** | **Yield stress (Pa)** | **Hysteresis Relative Area (\|Pa/s\|)** | **Re-dispersion after 1 month of storage (number of 180° rotation)** |
|---|---|---|---|---|
| **41** | -13.63 | 0.00 | 281.31 | > 30 (Sediment was redispersed only after vigorous hand shaking) |
| **55** | -21.3 | 1.64 | 190.95 | 10 |
| **56** | -17.6 | 1.81 | 84.18 | 5 |
| **57** | -13.5 | 0.19 | 43.39 | 3 |
| **58** | -10.57 | 0.68 | 26.172 | 3 |
| **59** | -7.46 | 0.00 | 2.26 | 2 |

As can be seen, the zeta potential values of the suspensions with 0.11% w/w SLS as wetting agent were all too high in terms of absolute value to allow stability of the suspensions. The increase of the concentration of pH adjusters (dihydrate sodium citrate + monohydrate citric acid) slightly diminished the absolute value of the zeta potential, which however was still too high. Based on these results, SLS was ruled out as wetting agent to be included in the powder mixture due to its inability to produce stable reconstituted liquid suspensions of reparixin.

Table 14 below reports the values of zeta potential and rheological properties obtained for the suspensions reported in reported in Table 7 above and of suspension **80** described above.

**Table 14**

| **Suspension** | **Zeta-Potential (mV)** | **Yield stress (Pa)** | **Hysteresis Relative Area (\|Pa/s\|)** | **Re-dispersion after 1 month of storage (number of 180° rotation)** |
|---|---|---|---|---|
| **68** | -1.30 | 0.84 | 48.173 | >20 (vigorous manual shaking is needed to resuspend the sediment |
| **69** | -1.60 | <0.01 | 3.20 | 20 |
| **70** | -1.10 | <0.01 | 45.91 | >20 (vigorous manual shaking is needed to resuspend the sediment |
| **71** | -1.10 | 0.40 | 86.88 | 10 |
| **72** | -0.96 | 0.00 | 21.70 | 8 |
| **78** | -1.26 | 5.09 | 99.69 | NA |
| **79** | -1.6 | 5.88 | 1205.70 | NA |
| **80** | -0.83 | 3.04 | 46.79 | Not-redispersible sediment |

As can be seen, all the suspensions reported in Table 7 were characterized by zeta potential values near zero. Based on these data, the inventors hypotesized that, surprisingly, the use of sucrose as sweetener could be involved in the achievement of this trend.

Without being bound to a theory or principle, this trend can be explained by an unexpected formation of a hydration layer of sucrose surrounding the suspended particles which allows to increase the distance between the ions (cations and anions) decreasing, in this way, the absolute value of the zeta-potential. This hydration layer ensures that, even in case a sediment is formed, it is easily resuspendable by manually shaking, since it is loose and not compact. The increase of the amount of the viscosity agent (Avicel CL-611) resulted in an improvement of the rheological properties. Indeed, suspensions **78** and **79,** comprising the highest amount of Avicel CL-611 among the suspensions reported in Table 7, were the ones characterized by the best rheological properties (higher yield stress and hysteresis area). Suspension **80,** although being characterized by suitable zeta potential and yield stress values, was characterized by the presence of a not-redispersable sediment.

Table 15 below reports the values of zeta potential and rheological properties obtained for the suspensions reported in Table 8 above.

**Table 15**

| **Suspension** | **Zeta-Potential (mV)** | **Yield stress (Pa)** | **Hysteresis Relative Area (\|Pa/s\|)** | **Re-dispersion after 1 month of storage (number of 180° rotation)** |
|---|---|---|---|---|
| **99** | -0.30 | 4.14 | 1542.2 | 5 (Persistent presence of floating foam at t0 and after 1 month as well) |
| **104** | This suspension was not characterized due to persistent presence of a floating foam (Reparixin was not completely wetted) | | | |
| **108** | -1.30 | 0.23 | 40.97 | NA (Persistent floating foam) |

As can be seen, when Avicel CL-611 was employed as viscosity agent in combination with PVP K30 as wetting agent, the resulting suspensions were characterized by a persisting foam on their surface, possibly due to the non complete wettability of reparixin. Based on these results, it was decided not to use Avicel CL-611 as viscosity agent and PVP as wetting agent in combination in the powder mixture according to the invention. Table 16 below reports the values of zeta potential and rheological properties obtained for the suspensions reported in Table 9 above.

**Table 16**

| **Suspension** | **Zeta-Potential (mV)** | **Yield stress (Pa)** | **Hysteresis Relative Area (\|Pa/s\|)** | **Re-dispersion after 1 month of storage (number of 180° rotation)** |
|---|---|---|---|---|
| **113** | -3.07 | 7.41 | 28.87 | 3 (Loose sediment-easy to re-disperse) |
| **120** | -1.40 | 7.41 | 9.10 | 2 (Loose sediment-easy to redisperse) |
| **131** | -2.43 | 4.54 | 2458 | NA (No sediment) |
| **133** | -2.06 | 3.15 | 592.30 | NA (No sediment) |

As can be seen, all the suspensions were characterized by suitable zeta potential values and rheological properties. Overall, suspensions **131** and **133** were characterized by the best properties.

The powder mixtures reported in Table 10 above were characterized in terms of flowability through Flodex by using Flodex Apparatus, Teledyne Hanson Research, Chatsworth, California, USA. This analysis is based upon the ability of powders to fall freely through a hole in a disc. For each substance a flowability index is reported, corresponding to the diameter of the hole in the disc (the scale is between 4 mm and 34 mm). The instrument is formed by a cylinder fixed to a shaft. The selected disc is secured to the cylinder. The hole in the disc is closed through a device. Approximately 50 g of powder are poured through a funnel into the middle of the disc. When loading is completed, 30 sec must be allowed to pass for possible formation of individual floccule or mass flocculation of the whole load. The device is then operated to open the hole in the disc quickly and without vibration. A very flowable powder will slowly flow through the small-diameter holes, leaving a cavity shaped like an upside-down, truncate cone. A powder with very low flowability, on the other hand, may come out of the hole or may fall abruptly. In this case the powder must be tested again with a disc with a larger hole.

Table 17 below reports the flodex value of the powder mixtures reported in Table 10 above.

**Table 17**

| **Formulation** | **Flodex (mm)** |
|---|---|
| **138** | 20 |
| **141** | 6 |
| **144** | 10 |
| **145** | 9 |

As can be seen from the above results, powder mixtures **141, 144** and **145** were characterized by suitable flodex values, suggesting that colloidal silica must be included in the powder mixture of the invention for achieving the desired flowability. Conversely, powder mixture **138,** where colloidal silica was absent, was characterized by a non-suitable flodex value.

Table 18 below reports the values of zeta potential and rheological properties obtained for the suspensions reported in Table 12 above, i.e., for the liquid suspensions reconstituted from the powder mixtures reported in Table 11 above.

**Table 18**

| **Suspension** | **Zeta-Potential (mV)** | **Yield stress (Pa)** | **Hysteresis Relative Area (\|Pa/s\|)** | **Appearance** |
|---|---|---|---|---|
| **163 lq** | -9.19 | 2.11 | 76.248 | Uniform appearance |
| **161 lq** | -3.09 | 1.40 | 100.01 | Uniform appearance |
| **141 lq** | -1.26 | 6.54 | 532.96 | No sediment |
| **162 lq** | -0.23 | 2.99 | 1553 | Uniform appearance. Presence of a layer of foam on the surface (barely perceptible). |
| **164 lq** | -0.09 | 12.04 | 7406.4 | Presence of foam on the surface. Very viscous suspension that makes handling and pouring impossible |
| **167 lq** | -12.5 | 2.78 | 73.213 | Presence of foam on the surface. Very viscous suspension that makes handling and pouring impossible |

This experiment was carried out in order to further investigate and confirm the impact of the type/amount of sweetener included in the formulation in the stabilization of zeta potential values of the reconstituted liquid suspensions. As can be seen, when 20% w/w sucrose was contained in the reconstituted liquid suspension (suspension **163 lq**), the absolute value of the zeta potential was too high, meaning that the suspension was not stable. The same trend was observed when 0.60% sucralose was contained in the reconstituted liquid suspension instead of sucrose (suspension **167 lq**).

When 60% w/w sucrose was contained in the reconstituted liquid suspension (suspension **164 lq**), the presence of a persistent layer of foam at the surface was observed, and the suspension was too viscous to be handled and poured.

When sucrose was contained in the reconstituted liquid suspensions in amounts of 30% w/w, 40% w/w and 50% w/w in the reconstituted suspensions (suspensions **161 lq, 141 lq** and **162 lq,** respectively), zeta potential values near zero and satisfactory rheological properties were obtained.

These results confirmed the unexpected impact of the type and amount of sweetener included in the powder mixture on the values of zeta potential and rheological properties of the reconstituted liquid suspension and, consequently, on the stability thereof. Sucrose was therefore selected as sweetener to be included in the powder mixture of the invention.

Table 19 below reports the values of zeta potential and rheological properties obtained for suspension **150 lq** reported in Table 12 above.

**Table 19**

| **Suspension** | **Zeta-Potential (mV)** | **Yield stress (Pa)** | **Hysteresis Relative Area (\|Pa/s\|)** | **Re-dispersion after 1 month of storage (number of 180° rotation)** |
|---|---|---|---|---|
| **150 Iq** | -0.812 | 9.927 | 1290.9 | NA (No sediment) |

The suspension was characterized by optimal zeta potential value and rheological properties. These data further confirmed the suitability of sucrose to stabilize the zeta potential value even in reconstituted liquid suspensions containing a higher amount of reparixin, particularly suitable for administration in adult subjects.

## Claims

1. A powder mixture comprising:
- reparixin in an amount between 3.5% w/w and 28.2% w/w,
- a viscosity agent selected from xanthan gum in an amount between 0.3% w/w and 0.5% w/w, gum arabic in an amount between 0.3% w/w and 0.5% w/w and a spray-dried blend of microcrystalline cellulose and sodium carboxymethylcellulose, wherein sodium carboxymethylcellulose is present in an amount between 11.3% w/w and 18.8% w/w, said blend being in an amount between 3% w/w and 8.3% w/w,
- sucrose in an amount between 63% w/w and 89% w/w,
- a wetting agent selected from polyvinylpyrrolidone in an amount between 3% w/w and 5% w/w, and poloxamer 188 in an amount between 0.5% w/w and 1.5% w/w,
- colloidal silica in an amount between 0.7% w/w and 1.4% w/w,
with the proviso that when said viscosity agent is said spray-dried blend of microcrystalline cellulose and sodium carboxymethylcellulose, said wetting agent is not polyvinylpyrrolidone.

2. The powder mixture according to claim 1, comprising:
- reparixin in an amount between 6.8% w/w and 28.2% w/w,
- a viscosity agent selected from xanthan gum in an amount between 0.3% w/w and 0.5% w/w, gum arabic in an amount between 0.3% w/w and 0.5% w/w, and a spray-dried blend of microcrystalline cellulose and sodium carboxymethylcellulose, wherein sodium carboxymethylcellulose is present in an amount between 11.3% w/w and 18.8% w/w, said blend being in an amount between 3% w/w and 8.3% w/w, preferably between 3% w/w and 8% w/w,
- sucrose in an amount between 63% w/w and 88% w/w,
- a wetting agent selected from polyvinylpyrrolidone in an amount between 3% w/w and 5% w/w and poloxamer 188 in an amount between 0.5% w/w and 1.5% w/w,
- colloidal silica in an amount between 0.7% w/w and 1.3% w/w,

3. The powder mixture according to claim 1 or claim 2, comprising:
- reparixin in an amount between amount between 7.6% w/w and 26.8% w/w,
- a viscosity agent selected from xanthan gum in an amount between 0.3% w/w and 0.5% w/w, gum arabic in an amount between 0.3% w/w and 0.5% w/w, and a spray-dried blend of microcrystalline cellulose and sodium carboxymethylcellulose, wherein sodium carboxymethylcellulose is present in an amount between 11.3% w/w and 18.8% w/w, said blend being in an amount between 4.5% w/w and 7.8% w/w,
- sucrose in an amount between 64.2% w/w and 85.2% w/w,
- a wetting agent selected from polyvinylpyrrolidone in an amount between 3% w/w and 5% w/w, and poloxamer 188 in an amount between 0.7% w/w and 1.3% w/w,
- colloidal silica in an amount between 0.7% w/w and 1.3% w/w.

4. The powder mixture according to any one of the preceding claims, comprising:
- reparixin in an amount between 8.4% w/w and 26% w/w,
- a viscosity agent selected from xanthan gum in an amount between 0.3% w/w and 0.5% w/w, gum arabic in an amount between 0.3% w/w and 0.5% w/w, and a spray-dried blend of microcrystalline cellulose and sodium carboxymethylcellulose, wherein sodium carboxymethylcellulose is present in an amount between 11.3% w/w and 18.8% w/w, said blend being in an amount between 4.5% w/w and 7.7% w/w,
- sucrose in an amount between 64.9% w/w and 84.5% w/w,
- a wetting agent selected from polyvinylpyrrolidone in an amount between 3% w/w and 5% w/w, and poloxamer 188 in an amount between 0.7% w/w and 1.3% w/w,
- colloidal silica in an amount between 0.7% w/w and 1.3% w/w.

5. The powder mixture according to any one of claims 1 to 3, comprising:
- reparixin in an amount between 7.6% w/w and 13.9% w/w,
- sucrose in an amount between 75.5% w/w and 85.2% w/w,
- a viscosity agent selected from xanthan gum in an amount between 0.3% w/w and 0.5% w/w, gum arabic in an amount between 0.3% w/w and 0.5% w/w and a spray-dried blend of microcrystalline cellulose and sodium carboxymethylcellulose, wherein sodium carboxymethylcellulose is present in an amount between 11.3% w/w and 18.8% w/w, said blend being in an amount between 5% w/w and 7.8% w/w,
- a wetting agent selected from polyvinylpyrrolidone in an amount between 3% w/w and 5% w/w, and poloxamer 188 in an amount between 0.8% w/w and 1.3% w/w,
- colloidal silica in an amout between 0.8% w/w and 1.3% w/w.

6. The powder mixture according to claim 1 or claim 2, comprising:
- reparixin in an amount between 17.5% w/w and 26.8% w/w,
- sucrose in an amount between 64.2% w/w and 76.1% w/w,
- a viscosity agent selected from xanthan gum in an amount between 0.3% w/w and 0.5% w/w, gum arabic in an amount between 0.3% w/w and 0.5% w/w and a spray-dried blend of microcrystalline cellulose and sodium carboxymethylcellulose, wherein sodium carboxymethylcellulose is present in an amount between 11.3% w/w and 18.8% w/w, said blend being in an amount between 4.5% w/w and 6.6% w/w,
- a wetting agent selected from polyvinylpyrrolidone in an amount between 3% w/w and 5% w/w, and poloxamer 188 in an amount between 0.7% w/w and 1.1% w/w,
- colloidal silica in an amount between 0.7% w/w and 1.1% w/w.

7. The powder mixture according to claim 1, constisting for at least 98%, preferably for at least 99% of its total weight, of:
- reparixin in an amount between 3.5% w/w and 28.2% w/w,
- a viscosity agent selected from xanthan gum in an amount between 0.3% w/w and 0.5% w/w, gum arabic in an amount between 0.3% w/w and 0.5% w/w and a spray-dried blend of microcrystalline cellulose and sodium carboxymethylcellulose, wherein sodium carboxymethylcellulose is present in an amount between 11.3% w/w and 18.8% w/w, said blend being in an amount between 3% w/w and 8.3% w/w,
- sucrose in an amount between 63% w/w and 89% w/w,
- a wetting agent selected from polyvinylpyrrolidone in an amount between 3% w/w and 5% w/w and poloxamer 188 in an amount between 0.5% w/w and 1.5% w/w,
- colloidal silica in an amount between 0.7% w/w and 1.4% w/w,
- a pH adjuster, preferably in an amount between 0.1% w/w and 0.2% w/w, and/or a preservative agent, preferably in an amount between 0.1% w/w and 0.5% w/w.

8. The powder mixture according to claim 2, constisting for at least 98%, preferably for at least 99% of its total weight, of:
- reparixin in an amount between 6.8% w/w and 28.2% w/w,
- a viscosity agent selected from xanthan gum in an amount between 0.3% w/w and 0.5% w/w, gum arabic in an amount between 0.3% w/w and 0.5% w/w, and a spray-dried blend of microcrystalline cellulose and sodium carboxymethylcellulose, wherein sodium carboxymethylcellulose is present in an amount between 11.3% w/w and 18.8% w/w, said blend being in an amount between 3% w/w and 8.3% w/w, preferably between 3% w/w and 8% w/w,
- sucrose in an amount between 63% w/w and 88% w/w,
- a wetting agent selected from polyvinylpyrrolidone in an amount between 3% w/w and 5% w/w and poloxamer 188 in an amount between 0.5% w/w and 1.5% w/w,
- colloidal silica in an amount between 0.7% w/w and 1.3% w/w,
- a pH adjuster, preferably in an amount between 0.1 w/w and 0.2% w/w, and/or a preservative agent, preferably in an amount between 0.1% w/w and 0.5% w/w.

9. The powder mixture according to claim 3, constisting for at least 98%, preferably for at least 99% of its total weight, of:
- reparixin in an amount between 7.6% w/w and 26.8% w/w,
- a viscosity agent selected from xanthan gum in an amount between 0.3% w/w and 0.5% w/w, gum arabic in an amount between 0.3% w/w and 0.5% w/w, and a spray-dried blend of microcrystalline cellulose and sodium carboxymethylcellulose, wherein sodium carboxymethylcellulose is present in an amount between 11.3% w/w and 18.8% w/w, said blend being in an amount between 4.5% w/w and 7.8% w/w,
- sucrose in an amount between 64.2% w/w and 85.2% w/w,
- a wetting agent selected from polyvinylpyrrolidone in an amount between 3% w/w and 5% w/w, and poloxamer 188 in an amount between 0.7% w/w and 1.3% w/w,
- colloidal silica in an amount between 0.7% w/w and 1.3% w/w
- a pH adjuster, preferably in an amount between 0.1% w/w and 0.2% w/w, and/or a preservative agent, preferably in an amount between 0.2% w/w and 0.4% w/w.

10. The powder mixture according to claim 4, constisting for at least 98%, preferably for at least 99% of its total weight, of:
- reparixin in an amount between 8.4% w/w and 26% w/w,
- a viscosity agent selected from xanthan gum in an amount between 0.3% w/w and 0.5% w/w, gum arabic in an amount between 0.3% w/w and 0.5% w/w, and a spray-dried blend of microcrystalline cellulose and sodium carboxymethylcellulose, wherein sodium carboxymethylcellulose is present in an amount between 11.3% w/w and 18.8% w/w, said blend being in an amount between 4.5% w/w and 7.7% w/w,
- sucrose in an amount between 64.9% w/w and 84.5% w/w,
- a wetting agent selected from polyvinylpyrrolidone in an amount between 3% w/w and 5% w/w, and poloxamer 188 in an amount between 0.7% w/w and 1.3% w/w,
- colloidal silica in an amount between 0.7% w/w and 1.3% w/w
- a pH adjuster, preferably in an amount between 0.1% w/w and 0.2% w/w, and/or a preservative agent, preferably in an amount between 0.2% w/w and 0.4% w/w.

11. The powder mixture according to claim 5, constisting for at least 98%, preferably for at least 99% of its total weight of:
- reparixin in an amount between 7.6% w/w and 13.9% w/w,
- sucrose in an amount between 75.5% w/w and 85.2% w/w,
- a preservative agent, preferably in an amount between 0.2% w/w and 0.4% w/w, and/or a pH adjuster, preferably in an amount between 0.10% w/w and 0.17% w/w,
- a viscosity agent selected from xanthan gum in an amount between 0.3% w/w and 0.5% w/w, gum arabic in an amount between 0.3% w/w and 0.5% w/w and a spray-dried blend of microcrystalline cellulose and sodium carboxymethylcellulose, wherein sodium carboxymethylcellulose is present in an amount between 11.3% w/w and 18.8% w/w, said blend being in an amount between 5% w/w and 7.8% w/w,
- a wetting agent selected from polyvinylpyrrolidone in an amount between 3% w/w and 5% w/w, and poloxamer 188 in an amount between 0.8% w/w and 1.3% w/w,
- colloidal silica in an amout between 0.8% w/w and 1.3% w/w.

12. The powder mixture according to claim 6, constisting for at least 98%, preferably for at least 99% of its total weight of:
- reparixin in an amount between 17.5% w/w and 26.8% w/w
- sucrose in an amount between 64.2% w/w and 76.1% w/w
- a preservative agent, preferably in an amount between 0.2% w/w and 0.35% w/w, and/or a pH adjuster, preferably in an amount between 0.1% w/w and 0.2% w/w,
- a viscosity agent selected from xanthan gum in an amount between 0.3% w/w and 0.5% w/w, gum arabic in an amount between 0.3% w/w and 0.5% w/w and a spray-dried blend of microcrystalline cellulose and sodium carboxymethylcellulose, wherein sodium carboxymethylcellulose is present in an amount between 11.3% w/w and 18.8% w/w, said blend being in an amount between 4.5% w/w and 6.6% w/w,
- a wetting agent selected from polyvinylpyrrolidone in an amount between 3% w/w and 5% w/w, and poloxamer 188 in an amount between 0.7% w/w and 1.1% w/w,
- colloidal silica in an amount between 0.7% w/w and 1.1% w/w.

13. The powder mixture according to any one of claims 7 to 12, wherein said preservative agent is selected from potassium sorbate, sodium benzoate, methyl parabens and propyl parabens, and/or said pH adjuster is selected from monohydrate citric acid, hydrochloric acid, sodium phosphate monobasic and potassium phosphate monobasic.

14. A process to produce a reconstituted liquid suspension from the powder mixture as claimed in any one of claims 1 to 13, including the step of suspending said powder mixture in an aqueous vehicle, preferably water.

15. The powder mixture according to any one of claims 1 to 13 or the reconstituted liquid suspension thereof for use in:
- the treatment of a disease or condition selected from psoriasis, rheumatoid arthritis, ulcerative colitis, acute respiratory distress syndrome (ARDS), idiopathic fibrosis, glomerulonephritis, COVID-19, pneumonia caused by SARS-CoV-2 or a variant thereof,
- the prevention of diabetes or in delaying the onset and the progression of diabetes, preferably Type I diabetes,
- the prevention or treatment of seizures in an individual.

## Patentansprüche

1. Pulvermischung, umfassend:
- Reparixin in einer Menge zwischen 3,5 Gew.- % und 28,2 Gew.- %,
- ein Viskositätsmittel, ausgewählt aus Xanthangummi in einer Menge zwischen 0,3 Gew.- % und 0,5 Gew.- %, Gummiarabikum in einer Menge zwischen 0,3 Gew.- % und 0,5 Gew.- % und einem sprühgetrockneten Gemisch aus mikrokristalliner Cellulose und Natriumcarboxymethylcellulose, wobei Natriumcarboxymethylcellulose in einer Menge zwischen 11,3 Gew.- % und 18,8 Gew.- % vorliegt, wobei das Gemisch in einer Menge zwischen 3 Gew.- % und 8,3 Gew.- % vorliegt,
- Saccharose in einer Menge zwischen 63 Gew.- % und 89 Gew.- %,
- ein Netzmittel ausgewählt aus Polyvinylpyrrolidon in einer Menge zwischen 3 Gew.-% und 5 Gew.- % und Poloxamer 188 in einer Menge zwischen 0,5 Gew.- % und 1,5 Gew.- %,
- kolloidales Siliziumdioxid in einer Menge zwischen 0,7 Gew.- % und 1,4 Gew.- %, mit der Maßgabe, dass, wenn es sich bei dem Viskositätsmittel um das sprühgetrocknete Gemisch aus mikrokristalliner Cellulose und Natriumcarboxymethylcellulose handelt, das Netzmittel nicht Polyvinylpyrrolidon ist.

2. Pulvermischung nach Anspruch 1, umfassend:
- Reparixin in einer Menge zwischen 6,8 Gew.- % und 28,2 Gew.- %,
- ein Viskositätsmittel, ausgewählt aus Xanthangummi in einer Menge zwischen 0,3 Gew.- % und 0,5 Gew.- %, Gummiarabikum in einer Menge zwischen 0,3 Gew.- % und 0,5 Gew.- % und einem sprühgetrockneten Gemisch aus mikrokristalliner Cellulose und Natriumcarboxymethylcellulose, wobei Natriumcarboxymethylcellulose in einer Menge zwischen 11,3 Gew.- % und 18,8 Gew.- % vorliegt, wobei das Gemisch in einer Menge zwischen 3 Gew.- % und 8,3 Gew.- %, vorzugsweise zwischen 3 Gew.- % und 8 Gew.- %, vorliegt,
- Saccharose in einer Menge zwischen 63 Gew.- % und 88 Gew.- %,
- ein Netzmittel, ausgewählt aus Polyvinylpyrrolidon in einer Menge zwischen 3 Gew.-% und 5 Gew.- % und Poloxamer 188 in einer Menge zwischen 0,5 Gew.- % und 1,5 Gew.- %,
- kolloidales Siliziumdioxid in einer Menge zwischen 0,7 Gew.- % und 1,3 Gew.- %,

3. Pulvermischung nach Anspruch 1 oder Anspruch 2, umfassend:
- Reparixin in einer Menge zwischen 7,6 Gew.- % und 26,8 Gew.- %,
- ein Viskositätsmittel, ausgewählt aus Xanthangummi in einer Menge zwischen 0,3 Gew.- % und 0,5 Gew.- %, Gummiarabikum in einer Menge zwischen 0,3 Gew.- % und 0,5 Gew.- % und einem sprühgetrockneten Gemisch aus mikrokristalliner Cellulose und Natriumcarboxymethylcellulose, wobei Natriumcarboxymethylcellulose in einer Menge zwischen 11,3 Gew.- % und 18,8 Gew.- % vorliegt, wobei das Gemisch in einer Menge zwischen 4,5 Gew.- % und 7,8 Gew.- % vorliegt,
- Saccharose in einer Menge zwischen 64,2 Gew.- % und 85,2 Gew.- %,
- ein Netzmittel, ausgewählt aus Polyvinylpyrrolidon in einer Menge zwischen 3 Gew.-% und 5 Gew.- % und Poloxamer 188 in einer Menge zwischen 0,7 Gew.- % und 1,3 Gew.- %,
- kolloidales Siliziumdioxid in einer Menge zwischen 0,7 Gew.- % und 1,3 Gew.- %.

4. Pulvermischung nach einem der vorhergehenden Ansprüche, umfassend:
- Reparixin in einer Menge zwischen 8,4 Gew.- % und 26 Gew.- %,
- ein Viskositätsmittel, ausgewählt aus Xanthangummi in einer Menge zwischen 0,3 Gew.- % und 0,5 Gew.- %, Gummiarabikum in einer Menge zwischen 0,3 Gew.- % und 0,5 Gew.- % und einem sprühgetrockneten Gemisch aus mikrokristalliner Cellulose und Natriumcarboxymethylcellulose, wobei Natriumcarboxymethylcellulose in einer Menge zwischen 11,3 Gew.- % und 18,8 Gew.- % vorliegt, wobei das Gemisch in einer Menge zwischen 4,5 Gew.- % und 7,7 Gew.- % vorliegt,
- Saccharose in einer Menge zwischen 64,9 Gew.- % und 84,5 Gew.- %,
- ein Netzmittel, ausgewählt aus Polyvinylpyrrolidon in einer Menge zwischen 3 Gew.-% und 5 Gew.- % und Poloxamer 188 in einer Menge zwischen 0,7 Gew.- % und 1,3 Gew.- %,
- kolloidales Siliziumdioxid in einer Menge zwischen 0,7 Gew.- % und 1,3 Gew.- %.

5. Pulvermischung nach einem der Ansprüche 1 bis 3, umfassend:
- Reparixin in einer Menge zwischen 7,6 Gew.- % und 13,9 Gew.- %,
- Saccharose in einer Menge zwischen 75,5 Gew.- % und 85,2 Gew.- %,
- ein Viskositätsmittel, ausgewählt aus Xanthangummi in einer Menge zwischen 0,3 Gew.- % und 0,5 Gew.- %, Gummiarabikum in einer Menge zwischen 0,3 Gew.- % und 0,5 Gew.- % und einem sprühgetrockneten Gemisch aus mikrokristalliner Cellulose und Natriumcarboxymethylcellulose, wobei Natriumcarboxymethylcellulose in einer Menge zwischen 11,3 Gew.- % und 18,8 Gew.- % vorliegt, wobei das Gemisch in einer Menge zwischen 5 Gew.- % und 7,8 Gew.- % vorliegt,
- ein Netzmittel, ausgewählt aus Polyvinylpyrrolidon in einer Menge zwischen 3 Gew.-% und 5 Gew.- % und Poloxamer 188 in einer Menge zwischen 0,8 Gew.- % und 1,3 Gew.- %,
- kolloidales Siliziumdioxid in einer Menge zwischen 0,8 Gew.- % und 1,3 Gew.- %.

6. Pulvermischung nach Anspruch 1 oder Anspruch 2, umfassend:
- Reparixin in einer Menge zwischen 17,5 Gew.- % und 26,8 Gew.- %,
- Saccharose in einer Menge zwischen 64,2 Gew.- % und 76,1 Gew.- %,
- ein Viskositätsmittel, ausgewählt aus Xanthangummi in einer Menge zwischen 0,3 Gew.- % und 0,5 Gew.- %, Gummiarabikum in einer Menge zwischen 0,3 Gew.- % und 0,5 Gew.- % und einem sprühgetrockneten Gemisch aus mikrokristalliner Cellulose und Natriumcarboxymethylcellulose, wobei Natriumcarboxymethylcellulose in einer Menge zwischen 11,3 Gew.- % und 18,8 Gew.- % vorliegt, wobei das Gemisch in einer Menge zwischen 4,5 Gew.- % und 6,6 Gew.- % vorliegt,
- ein Netzmittel, ausgewählt aus Polyvinylpyrrolidon in einer Menge zwischen 3 Gew.-% und 5 Gew.- % und Poloxamer 188 in einer Menge zwischen 0,7 Gew.- % und 1,1 Gew.- %,
- kolloidales Siliziumdioxid in einer Menge zwischen 0,7 Gew.- % und 1,1 Gew.- %.

7. Pulvermischung nach Anspruch 1, bestehend zu mindestens 98 %, vorzugsweise zu mindestens 99 % ihres Gesamtgewichts aus:
- Reparixin in einer Menge zwischen 3,5 Gew.- % und 28,2 Gew.- %,
- einem Viskositätsmittel, ausgewählt aus Xanthangummi in einer Menge zwischen 0,3 Gew.- % und 0,5 Gew.- %, Gummiarabikum in einer Menge zwischen 0,3 Gew.- % und 0,5 Gew.- % und einem sprühgetrockneten Gemisch aus mikrokristalliner Cellulose und Natriumcarboxymethylcellulose, wobei Natriumcarboxymethylcellulose in einer Menge zwischen 11,3 Gew.- % und 18,8 Gew.- % vorliegt, wobei das Gemisch in einer Menge zwischen 3 Gew.- % und 8,3 Gew.- % vorliegt,
- Saccharose in einer Menge zwischen 63 Gew.- % und 89 Gew.- %,
- einem Netzmittel, ausgewählt aus Polyvinylpyrrolidon in einer Menge zwischen 3 Gew.- % und 5 Gew.- % und Poloxamer 188 in einer Menge zwischen 0,5 Gew.- % und 1,5 Gew.- %,
- kolloidalem Siliziumdioxid in einer Menge zwischen 0,7 Gew.- % und 1,4 Gew.- %,
- einem pH-Regulator, vorzugsweise in einer Menge zwischen 0,1 Gew.- % und 0,2 Gew.- %, und/oder einem Konservierungsmittel, vorzugsweise in einer Menge zwischen 0,1 Gew.- % und 0,5 Gew.- %.

8. Pulvermischung nach Anspruch 2, bestehend zu mindestens 98 %, vorzugsweise zu mindestens 99 % ihres Gesamtgewichts aus:
- Reparixin in einer Menge zwischen 6,8 Gew.- % und 28,2 Gew.- %,
- einem Viskositätsmittel, ausgewählt aus Xanthangummi in einer Menge zwischen 0,3 Gew.- % und 0,5 Gew.- %, Gummiarabikum in einer Menge zwischen 0,3 Gew.- % und 0,5 Gew.- % und einem sprühgetrockneten Gemisch aus mikrokristalliner Cellulose und Natriumcarboxymethylcellulose, wobei Natriumcarboxymethylcellulose in einer Menge zwischen 11,3 Gew.- % und 18,8 Gew.- % vorliegt, wobei das Gemisch in einer Menge zwischen 3 Gew.- % und 8,3 Gew.- %, vorzugsweise zwischen 3 Gew.- % und 8 Gew.- %, vorliegt,
- Saccharose in einer Menge zwischen 63 Gew.- % und 88 Gew.- %,
- einem Netzmittel, ausgewählt aus Polyvinylpyrrolidon in einer Menge zwischen 3 Gew.- % und 5 Gew.- % und Poloxamer 188 in einer Menge zwischen 0,5 Gew.- % und 1,5 Gew.- %,
- kolloidalem Siliziumdioxid in einer Menge zwischen 0,7 Gew.- % und 1,3 Gew.- %,
- einem pH-Regulator, vorzugsweise in einer Menge zwischen 0,1 Gew.- % und 0,2 Gew.- %, und/oder einem Konservierungsmittel, vorzugsweise in einer Menge zwischen 0,1 Gew.- % und 0,5 Gew.- %.

9. Pulvermischung nach Anspruch 3, bestehend zu mindestens 98 %, vorzugsweise zu mindestens 99 % ihres Gesamtgewichts aus:
- Reparixin in einer Menge zwischen 7,6 Gew.- % und 26,8 Gew.- %,
- einem Viskositätsmittel, ausgewählt aus Xanthangummi in einer Menge zwischen 0,3 Gew.- % und 0,5 Gew.- %, Gummiarabikum in einer Menge zwischen 0,3 Gew.- % und 0,5 Gew.- % und einem sprühgetrockneten Gemisch aus mikrokristalliner Cellulose und Natriumcarboxymethylcellulose, wobei Natriumcarboxymethylcellulose in einer Menge zwischen 11,3 Gew.- % und 18,8 Gew.- % vorliegt, wobei das Gemisch in einer Menge zwischen 4,5 Gew.- % und 7,8 Gew.- % vorliegt,
- Saccharose in einer Menge zwischen 64,2 Gew.- % und 85,2 Gew.- %,
- einem Netzmittel, ausgewählt aus Polyvinylpyrrolidon in einer Menge zwischen 3 Gew.- % und 5 Gew.- % und Poloxamer 188 in einer Menge zwischen 0,7 Gew.- % und 1,3 Gew.- %,
- kolloidalem Siliziumdioxid in einer Menge zwischen 0,7 Gew.- % und 1,3 Gew.- %.
- einem pH-Regulator, vorzugsweise in einer Menge zwischen 0,1 Gew.- % und 0,2 Gew.- %, und/oder einem Konservierungsmittel, vorzugsweise in einer Menge zwischen 0,2 Gew.- % und 0,4 Gew.- %.

10. Pulvermischung nach Anspruch 4, bestehend zu mindestens 98 %, vorzugsweise zu mindestens 99 % ihres Gesamtgewichts aus:
- Reparixin in einer Menge zwischen 8,4 Gew.- % und 26 Gew.- %,
- einem Viskositätsmittel, ausgewählt aus Xanthangummi in einer Menge zwischen 0,3 Gew.- % und 0,5 Gew.- %, Gummiarabikum in einer Menge zwischen 0,3 Gew.- % und 0,5 Gew.- % und einem sprühgetrockneten Gemisch aus mikrokristalliner Cellulose und Natriumcarboxymethylcellulose, wobei Natriumcarboxymethylcellulose in einer Menge zwischen 11,3 Gew.- % und 18,8 Gew.- % vorliegt, wobei das Gemisch in einer Menge zwischen 4,5 Gew.- % und 7,7 Gew.- % vorliegt,
- Saccharose in einer Menge zwischen 64,9 Gew.- % und 84,5 Gew.- %,
- einem Netzmittel, ausgewählt aus Polyvinylpyrrolidon in einer Menge zwischen 3 Gew.- % und 5 Gew.- % und Poloxamer 188 in einer Menge zwischen 0,7 Gew.- % und 1,3 Gew.- %,
- kolloidalem Siliziumdioxid in einer Menge zwischen 0,7 Gew.- % und 1,3 Gew.- %.
- einem pH-Regulator, vorzugsweise in einer Menge zwischen 0,1 Gew.- % und 0,2 Gew.- %, und/oder einem Konservierungsmittel, vorzugsweise in einer Menge zwischen 0,2 Gew.- % und 0,4 Gew.- %.

11. Pulvermischung nach Anspruch 5, bestehend zu mindestens 98 %, vorzugsweise zu mindestens 99 % ihres Gesamtgewichts aus:
- Reparixin in einer Menge zwischen 7,6 Gew.- % und 13,9 Gew.- %,
- Saccharose in einer Menge zwischen 75,5 Gew.- % und 85,2 Gew.- %,
- einem Konservierungsmittel, vorzugsweise in einer Menge zwischen 0,2 Gew.- % und 0,4 Gew.- %, und/oder einem pH-Regulator, vorzugsweise in einer Menge zwischen 0,10 Gew.- % und 0,17 Gew.- %,
- einem Viskositätsmittel, ausgewählt aus Xanthangummi in einer Menge zwischen 0,3 Gew.- % und 0,5 Gew.- %, Gummiarabikum in einer Menge zwischen 0,3 Gew.- % und 0,5 Gew.- % und einem sprühgetrockneten Gemisch aus mikrokristalliner Cellulose und Natriumcarboxymethylcellulose, wobei Natriumcarboxymethylcellulose in einer Menge zwischen 11,3 Gew.- % und 18,8 Gew.- % vorliegt, wobei das Gemisch in einer Menge zwischen 5 Gew.- % und 7,8 Gew.- % vorliegt,
- einem Netzmittel, ausgewählt aus Polyvinylpyrrolidon in einer Menge zwischen 3 Gew.- % und 5 Gew.- % und Poloxamer 188 in einer Menge zwischen 0,8 Gew.- % und 1,3 Gew.- %,
- kolloidalem Siliziumdioxid in einer Menge zwischen 0,8 Gew.- % und 1,3 Gew.- %.

12. Pulvermischung nach Anspruch 6, bestehend zu mindestens 98 %, vorzugsweise zu mindestens 99 % ihres Gesamtgewichts aus:
- Reparixin in einer Menge zwischen 17,5 Gew.- % und 26,8 Gew.- %,
- Saccharose in einer Menge zwischen 64,2 Gew.- % und 76,1 Gew.- %
- einem Konservierungsmittel, vorzugsweise in einer Menge zwischen 0,2 Gew.- % und 0,35 Gew.- %, und/oder einem pH-Regulator, vorzugsweise in einer Menge zwischen 0,1 Gew.- % und 0,2 Gew.- %,
- einem Viskositätsmittel, ausgewählt aus Xanthangummi in einer Menge zwischen 0,3 Gew.- % und 0,5 Gew.- %, Gummiarabikum in einer Menge zwischen 0,3 Gew.- % und 0,5 Gew.- % und einem sprühgetrockneten Gemisch aus mikrokristalliner Cellulose und Natriumcarboxymethylcellulose, wobei Natriumcarboxymethylcellulose in einer Menge zwischen 11,3 Gew.- % und 18,8 Gew.- % vorliegt, wobei das Gemisch in einer Menge zwischen 4,5 Gew.- % und 6,6 Gew.- % vorliegt,
- einem Netzmittel, ausgewählt aus Polyvinylpyrrolidon in einer Menge zwischen 3 Gew.- % und 5 Gew.- % und Poloxamer 188 in einer Menge zwischen 0,7 Gew.- % und 1,1 Gew.- %,
- kolloidalem Siliziumdioxid in einer Menge zwischen 0,7 Gew.- % und 1,1 Gew.- %.

13. Pulvermischung nach einem der Ansprüche 7 bis 12, wobei das Konservierungsmittel aus Kaliumsorbat, Natriumbenzoat, Methylparabenen und Propylparabenen ausgewählt ist und/oder der pH-Regulator aus Monohydratzitronensäure, Salzsäure, einbasischem Natriumphosphat und einbasischem Kaliumphosphat ausgewählt ist.

14. Verfahren zur Herstellung einer rekonstituierten flüssigen Suspension aus der Pulvermischung nach einem der Ansprüche 1 bis 13, umfassend den Schritt des Suspendierens der Pulvermischung in einem wässrigen Vehikel, vorzugsweise Wasser.

15. Pulvermischung nach einem der Ansprüche 1 bis 13 oder die rekonstituierte flüssige Suspension davon zur Verwendung bei:
- der Behandlung einer Erkrankung oder eines Zustands, ausgewählt aus Psoriasis, rheumatoider Arthritis, Colitis ulcerosa, akutem Atemnotsyndrom (ARDS), idiopathischer Fibrose, Glomerulonephritis, COVID-19, einer durch SARS-CoV-2 oder eine Variante davon verursachten Lungenentzündung,
- der Vorbeugung von Diabetes oder zur Verzögerung des Ausbruchs und des Fortschreitens von Diabetes, vorzugsweise Typ-I-Diabetes,
- der Vorbeugung oder Behandlung von Anfällen bei einer Person.

## Revendications

1. Mélange de poudres comprenant :
- de la réparixine en une quantité comprise entre 3,5 % p/p et 28,2 % p/p,
- un agent de viscosité choisi parmi la gomme xanthane, en une quantité comprise entre 0,3 % p/p et 0,5 % p/p, de la gomme arabique en une quantité comprise entre 0,3 % p/p et 0,5 % p/p, et d'un mélange séché par atomisation de cellulose microcristalline et de carboxyméthylcellulose sodique, dans lequel la carboxyméthylcellulose sodique est présente en une quantité comprise entre 11,3 % p/p et 18,8 % p/p, ledit mélange étant présent en une quantité comprise entre 3 % p/p et 8,3 % p/p,
- du saccharose en une quantité comprise entre 63 % p/p et 89 % p/p,
- un agent mouillant choisi parmi la polyvinylpyrrolidone, en une quantité comprise entre 3 % p/p et 5 % p/p, et le poloxamère 188, en une quantité comprise entre 0,5 % p/p et 1,5 % p/p,
- de la silice colloïdale en une quantité comprise entre 0,7 % p/p et 1,4 % p/p,
à condition que, lorsque ledit agent de viscosité est ledit mélange séché par atomisation de cellulose microcristalline et de carboxyméthylcellulose sodique, ledit agent mouillant ne soit pas de la polyvinylpyrrolidone.

2. Mélange de poudres selon la revendication 1, comprenant :
- de la réparixine en une quantité comprise entre 6,8 % p/p et 28,2 % p/p,
- un agent de viscosité choisi parmi la gomme xanthane, en une quantité comprise entre 0,3 % p/p et 0,5 % p/p, de la gomme arabique en une quantité comprise entre 0,3 % p/p et 0,5 % p/p, et un mélange séché par atomisation de cellulose microcristalline et de carboxyméthylcellulose sodique, dans lequel la carboxyméthylcellulose sodique est présente en une quantité comprise entre 11,3 % p/p et 18,8 % p/p, ledit mélange étant présent en une quantité comprise entre 3 % p/p et 8,3 % p/p, de préférence entre 3 % p/p et 8 % p/p,
- du saccharose en une quantité comprise entre 63 % p/p et 88 % p/p,
- un agent mouillant choisi parmi la polyvinylpyrrolidone, en une quantité comprise entre 3 % p/p et 5 % p/p, et le poloxamère 188, en une quantité comprise entre 0,5 % p/p et 1,5 % p/p,
- de la silice colloïdale en une quantité comprise entre 0,7 % p/p et 1,3 % p/p,

3. Mélange de poudres selon la revendication 1 ou la revendication 2, comprenant :
- de la réparixine en une quantité comprise entre 7,6 % p/p et 26,8 % p/p,
- un agent de viscosité choisi parmi la gomme xanthane, en une quantité comprise entre 0,3 % p/p et 0,5 % p/p, de la gomme arabique en une quantité comprise entre 0,3 % p/p et 0,5 % p/p, et un mélange séché par atomisation de cellulose microcristalline et de carboxyméthylcellulose sodique, dans lequel la carboxyméthylcellulose sodique est présente en une quantité comprise entre 11,3 % p/p et 18,8 % p/p, ledit mélange étant présent en une quantité comprise entre 4,5 % p/p et 7,8 % p/p,
- du saccharose en une quantité comprise entre 64,2 % p/p et 85,2 % p/p,
- un agent mouillant choisi parmi la polyvinylpyrrolidone, en une quantité comprise entre 3 % p/p et 5 % p/p, et le poloxamère 188, en une quantité comprise entre 0,7 % p/p et 1,3 % p/p,
- de la silice colloïdale en une quantité comprise entre 0,7 % p/p et 1,3 % p/p.

4. Mélange de poudres selon l'une quelconque des revendications précédentes, comprenant :
- de la réparixine en une quantité comprise entre 8,4 % p/p et 26 % p/p,
- un agent de viscosité choisi parmi la gomme xanthane, en une quantité comprise entre 0,3 % p/p et 0,5 % p/p, de la gomme arabique en une quantité comprise entre 0,3 % p/p et 0,5 % p/p, et un mélange séché par atomisation de cellulose microcristalline et de carboxyméthylcellulose sodique, dans lequel la carboxyméthylcellulose sodique est présente en une quantité comprise entre 11,3 % p/p et 18,8 % p/p, ledit mélange étant présent en une quantité comprise entre 4,5 % p/p et 7,7 % p/p,
- du saccharose en une quantité comprise entre 64,9 % p/p et 84,5 % p/p,
- un agent mouillant choisi parmi la polyvinylpyrrolidone, en une quantité comprise entre 3 % p/p et 5 % p/p, et le poloxamère 188, en une quantité comprise entre 0,7 % p/p et 1,3 % p/p,
- de la silice colloïdale en une quantité comprise entre 0,7 % p/p et 1,3 % p/p.

5. Mélange de poudres selon l'une quelconque des revendications 1 à 3, comprenant :
- de la réparixine en une quantité comprise entre 7,6 % p/p et 13,9 % p/p,
- du saccharose en une quantité comprise entre 75,5 % p/p et 85,2 % p/p,
- un agent de viscosité choisi parmi la gomme xanthane, en une quantité comprise entre 0,3 % p/p et 0,5 % p/p, de la gomme arabique en une quantité comprise entre 0,3 % p/p et 0,5 % p/p, et d'un mélange séché par atomisation de cellulose microcristalline et de carboxyméthylcellulose sodique, dans lequel la carboxyméthylcellulose sodique est présente en une quantité comprise entre 11,3 % p/p et 18,8 % p/p, ledit mélange étant présent en une quantité comprise entre 5 % p/p et 7,8 % p/p,
- un agent mouillant choisi parmi la polyvinylpyrrolidone, en une quantité comprise entre 3 % p/p et 5 % p/p, et le poloxamère 188, en une quantité comprise entre 0,8 % p/p et 1,3 % p/p,
- de la silice colloïdale en une quantité comprise entre 0,8 % p/p et 1,3 % p/p.

6. Mélange de poudres selon la revendication 1 ou la revendication 2, comprenant :
- de la réparixine en une quantité comprise entre 17,5 % p/p et 26,8 % p/p,
- du saccharose en une quantité comprise entre 64,2 % p/p et 76,1 % p/p,
- un agent de viscosité choisi parmi la gomme xanthane, en une quantité comprise entre 0,3 % p/p et 0,5 % p/p, de la gomme arabique en une quantité comprise entre 0,3 % p/p et 0,5 % p/p, et d'un mélange séché par atomisation de cellulose microcristalline et de carboxyméthylcellulose sodique, dans lequel la carboxyméthylcellulose sodique est présente en une quantité comprise entre 11,3 % p/p et 18,8 % p/p, ledit mélange étant présent en une quantité comprise entre 4,5 % p/p et 6,6 % p/p,
- un agent mouillant choisi parmi la polyvinylpyrrolidone, en une quantité comprise entre 3 % p/p et 5 % p/p, et le poloxamère 188, en une quantité comprise entre 0,7 % p/p et 1,1 % p/p,
- de la silice colloïdale en une quantité comprise entre 0,7 % p/p et 1,1 % p/p.

7. Mélange de poudres selon la revendication 1, constitué d'au moins 98 %, de préférence d'au moins 99 % de son poids total, de :
- réparixine en une quantité comprise entre 3,5 % p/p et 28,2 % p/p,
- un agent de viscosité choisi parmi la gomme xanthane, en une quantité comprise entre 0,3 % p/p et 0,5 % p/p, de la gomme arabique en une quantité comprise entre 0,3 % p/p et 0,5 % p/p, et d'un mélange séché par atomisation de cellulose microcristalline et de carboxyméthylcellulose sodique, dans lequel la carboxyméthylcellulose sodique est présente en une quantité comprise entre 11,3 % p/p et 18,8 % p/p, ledit mélange étant présent en une quantité comprise entre 3 % p/p et 8,3 % p/p,
- saccharose en une quantité comprise entre 63 % p/p et 89 % p/p,
- un agent mouillant choisi parmi la polyvinylpyrrolidone, en une quantité comprise entre 3 % p/p et 5 % p/p, et le poloxamère 188, en une quantité comprise entre 0,5 % p/p et 1,5 % p/p,
- silice colloïdale en une quantité comprise entre 0,7 % p/p et 1,4 % p/p,
- un régulateur de pH, de préférence en une quantité comprise entre 0,1 % p/p et 0,2 % p/p, et/ou un agent conservateur, de préférence en une quantité comprise entre 0,1 % p/p et 0,5 % p/p.

8. Mélange de poudres selon la revendication 2, constitué d'au moins 98 %, de préférence d'au moins 99 % de son poids total, de :
- réparixine en une quantité comprise entre 6,8 % p/p et 28,2 % p/p,
- un agent de viscosité choisi parmi la gomme xanthane, en une quantité comprise entre 0,3 % p/p et 0,5 % p/p, de la gomme arabique en une quantité comprise entre 0,3 % p/p et 0,5 % p/p, et un mélange séché par atomisation de cellulose microcristalline et de carboxyméthylcellulose sodique, dans lequel la carboxyméthylcellulose sodique est présente en une quantité comprise entre 11,3 % p/p et 18,8 % p/p, ledit mélange étant présent en une quantité comprise entre 3 % p/p et 8,3 % p/p, de préférence entre 3 % p/p et 8 % p/p,
- saccharose en une quantité comprise entre 63 % p/p et 88 % p/p,
- un agent mouillant choisi parmi la polyvinylpyrrolidone, en une quantité comprise entre 3 % p/p et 5 % p/p, et le poloxamère 188, en une quantité comprise entre 0,5 % p/p et 1,5 % p/p,
- silice colloïdale en une quantité comprise entre 0,7 % p/p et 1,3 % p/p,
- un régulateur de pH, de préférence en une quantité comprise entre 0,1 % p/p et 0,2 % p/p, et/ou un agent conservateur, de préférence en une quantité comprise entre 0,1 % p/p et 0,5 % p/p.

9. Mélange de poudres selon la revendication 3, constitué d'au moins 98 %, de préférence d'au moins 99 % de son poids total, de :
- réparixine en une quantité comprise entre 7,6 % p/p et 26,8 % p/p,
- un agent de viscosité choisi parmi la gomme xanthane, en une quantité comprise entre 0,3 % p/p et 0,5 % p/p, de la gomme arabique en une quantité comprise entre 0,3 % p/p et 0,5 % p/p, et un mélange séché par atomisation de cellulose microcristalline et de carboxyméthylcellulose sodique, dans lequel la carboxyméthylcellulose sodique est présente en une quantité comprise entre 11,3 % p/p et 18,8 % p/p, ledit mélange étant présent en une quantité comprise entre 4,5 % p/p et 7,8 % p/p,
- saccharose en une quantité comprise entre 64,2 % p/p et 85,2 % p/p,
- un agent mouillant choisi parmi la polyvinylpyrrolidone, en une quantité comprise entre 3 % p/p et 5 % p/p, et le poloxamère 188, en une quantité comprise entre 0,7 % p/p et 1,3 % p/p,
- silice colloïdale en une quantité comprise entre 0,7 % p/p et 1,3 % p/p
- un régulateur de pH, de préférence en une quantité comprise entre 0,1 % p/p et 0,2 % p/p, et/ou un agent conservateur, de préférence en une quantité comprise entre 0,2 % p/p et 0,4 % p/p.

10. Mélange de poudres selon la revendication 4, constitué d'au moins 98 %, de préférence d'au moins 99 % de son poids total, de :
- réparixine en une quantité comprise entre 8,4 % p/p et 26 % p/p,
- un agent de viscosité choisi parmi la gomme xanthane, en une quantité comprise entre 0,3 % p/p et 0,5 % p/p, de la gomme arabique en une quantité comprise entre 0,3 % p/p et 0,5 % p/p, et un mélange séché par atomisation de cellulose microcristalline et de carboxyméthylcellulose sodique, dans lequel la carboxyméthylcellulose sodique est présente en une quantité comprise entre 11,3 % p/p et 18,8 % p/p, ledit mélange étant présent en une quantité comprise entre 4,5 % p/p et 7,7 % p/p,
- saccharose en une quantité comprise entre 64,9 % p/p et 84,5 % p/p,
- un agent mouillant choisi parmi la polyvinylpyrrolidone, en une quantité comprise entre 3 % p/p et 5 % p/p, et le poloxamère 188, en une quantité comprise entre 0,7 % p/p et 1,3 % p/p,
- silice colloïdale en une quantité comprise entre 0,7 % p/p et 1,3 % p/p
- un régulateur de pH, de préférence en une quantité comprise entre 0,1 % p/p et 0,2 % p/p, et/ou un agent conservateur, de préférence en une quantité comprise entre 0,2 % p/p et 0,4 % p/p.

11. Mélange de poudres selon la revendication 5, constitué d'au moins 98 %, de préférence d'au moins 99 % de son poids total, de :
- réparixine en une quantité comprise entre 7,6 % p/p et 13,9 % p/p,
- saccharose en une quantité comprise entre 75,5 % p/p et 85,2 % p/p,
- un agent conservateur, de préférence en une quantité comprise entre 0,2 % p/p et 0,4 % p/p, et/ou un régulateur de pH, de préférence en une quantité comprise entre 0,10 % p/p et 0,17 % p/p,
- un agent de viscosité choisi parmi la gomme xanthane, en une quantité comprise entre 0,3 % p/p et 0,5 % p/p, de la gomme arabique en une quantité comprise entre 0,3 % p/p et 0,5 % p/p, et d'un mélange séché par atomisation de cellulose microcristalline et de carboxyméthylcellulose sodique, dans lequel la carboxyméthylcellulose sodique est présente en une quantité comprise entre 11,3 % p/p et 18,8 % p/p, ledit mélange étant présent en une quantité comprise entre 5 % p/p et 7,8 % p/p,
- un agent mouillant choisi parmi la polyvinylpyrrolidone, en une quantité comprise entre 3 % p/p et 5 % p/p, et le poloxamère 188, en une quantité comprise entre 0,8 % p/p et 1,3 % p/p,
- silice colloïdale en une quantité comprise entre 0,8 % p/p et 1,3 % p/p.

12. Mélange de poudres selon la revendication 6, constitué d'au moins 98 %, de préférence d'au moins 99 % de son poids total, de :
- réparixine en une quantité comprise entre 17,5 % p/p et 26,8 % p/p
- saccharose en une quantité comprise entre 64,2 % p/p et 76,1 % p/p
- un agent conservateur, de préférence en une quantité comprise entre 0,2 % p/p et 0,35 % p/p, et/ou un régulateur de pH, de préférence en une quantité comprise entre 0,1 % p/p et 0,2 % p/p,
- un agent de viscosité choisi parmi la gomme xanthane, en une quantité comprise entre 0,3 % p/p et 0,5 % p/p, de la gomme arabique en une quantité comprise entre 0,3 % p/p et 0,5 % p/p, et d'un mélange séché par atomisation de cellulose microcristalline et de carboxyméthylcellulose sodique, dans lequel la carboxyméthylcellulose sodique est présente en une quantité comprise entre 11,3 % p/p et 18,8 % p/p, ledit mélange étant présent en une quantité comprise entre 4,5 % p/p et 6,6 % p/p,
- un agent mouillant choisi parmi la polyvinylpyrrolidone, en une quantité comprise entre 3 % p/p et 5 % p/p, et le poloxamère 188, en une quantité comprise entre 0,7 % p/p et 1,1 % p/p,
- silice colloïdale en une quantité comprise entre 0,7 % p/p et 1,1 % p/p.

13. Mélange de poudres selon l'une quelconque des revendications 7 à 12, dans lequel ledit agent conservateur est choisi parmi le sorbate de potassium, du benzoate de sodium, des parabènes de méthyle et des parabènes de propyle, et/ou dans lequel ledit régulateur de pH est choisi parmi l'acide citrique monohydraté, l'acide chlorhydrique, le phosphate monobasique de sodium et le phosphate monobasique de potassium.

14. Procédé de préparation d'une suspension liquide reconstituée à partir du mélange de poudres selon l'une quelconque des revendications 1 à 13, comprenant l'étape consistant à mettre en suspension ledit mélange de poudres dans un véhicule aqueux, de préférence de l'eau.

15. Mélange de poudres selon l'une quelconque des revendications 1 à 13, ou la suspension liquide reconstituée de celui-ci, destiné à être utilisé dans :
- le traitement d'une maladie ou d'un trouble choisi parmi le psoriasis, la polyarthrite rhumatoïde, la colite ulcéreuse, le syndrome de détresse respiratoire aiguë (SDRA), la fibrose idiopathique, la glomérulonéphrite, la COVID-19, la pneumonie causée par le SARS-CoV-2 ou l'un de ses variants,
- la prévention du diabète ou le retardement de son apparition et de sa progression, de préférence du diabète de type I,
- la prévention ou le traitement des crises chez un individu.
